# FASCICULE DE BREVET EUROPEEN

(11) **EP 1 404 816 B1**
(45) Date de publication et mention de la délivrance du brevet: **17.02.2010**
(21) Numéro de dépôt: 02764950.8
(22) Date de dépôt: 08.07.2002
(51) Int. Cl.: C12N 5/09, C12N 5/02, C12N 15/63, C12Q 1/68, C12Q 1/70, C12Q 1/02, C07K 16/08, A61K 39/12, A61K 35/407, A61K 35/76, A61P 31/12

(54) **LIGNEES D'HEPATOMES HUMAINS LEURS OBTENTION ET LEURS APPLICATIONS**
MENSCHLICHE HEPATOMAZELLINIEN, DEREN GEWINUNG UND DEREN VERWENDUNGEN
HUMAN HEPATOMA CELL LINES, METHODS FOR OBTAINING SAME AND USES THEREOF

(30) Priorité: 06.07.2001 FR 0109044
(43) Date de publication de la demande: 07.04.2004
(73) Titulaire: INSTITUT NATIONAL DE LA SANTE ET DE LA RECHERCHE MEDICALE (INSERM), 75654 Paris Cédex 13 (FR)
(72) Inventeur: GRIPON, Philippe, F-35000 Rennes (FR); RUMIN, Sylvie, 35230 Orgeres (FR); GUGUEN-GUILLOUZO, Christiane, F-35000 Rennes (FR); TREPO, Christian, F-69500 Bron (FR)
(74) Mandataire: Peaucelle, Chantal
(86) Numéro de dépôt international: PCT/FR2002/002391
(87) Numéro de publication internationale: WO 2003/004627

(56) Documents cités:
- EP-A- 0 972 828
- WO-A-94/08598
- WO-A-96/30492
- US-A- 4 393 133
- US-A- 5 804 441
- NEURATH ET AL: "SEARCH FOR HEPATITIS B VIRUS CELL RECEPTORS REVEALS BINDING SITES FOR INTERLEUKIN 6 ON THE VIRUS ENVELOPE PROTEIN" JOURNAL OF EXPERIMENTAL MEDICINE, TOKYO, JP, no. 175, 1992, pages 461-469, XP002076255 ISSN: 0022-1007
- CHIU ET AL.: "The formation of bile canaliculi in human hepatoma cell lines" HEPATOLOGY, vol. 11, no. 5, mai 1990 (1990-05), pages 834-842, XP008013762
- LIAN ET AL.: "Targeting of aminopeptidase N to bile caniculi correlates with secretory activities of the developing canalicular domain" HEPATOLOGY, vol. 30, no. 3, septembre 1999 (1999-09), pages 748-760, XP008013737
- GRIPON P ET AL: "REGULATION BY DIMETHYLSULFOXIDE, INSULIN, AND CORTICOSTEROIDS OF HEPATITIS B VIRUS REPLICATION IN A TRANSFECTED HUMAN HEPATOMA CELL LINE" JOURNAL OF MEDICAL VIROLOGY, NEW YORK, NY, US, vol. 28, 1989, pages 193-199, XP000872077
- SAITO ET AL.: "Effect of dexamethasone, dimethylsulfoxide and sodium butyrate on a human hepatoma cell line PLC/PRF/5" CANCER BIOCHEMISTRY BIPHYSICS, vol. 13, no. 2, novembre 1992 (1992-11), pages 75-84, XP008001762
- ZVIBEL I. ET AL: 'Phenotypic characterization of rat hepatoma cell lines and lineage-specific regulation of gene expression by differentiation agents' DIFFERENTIATION vol. 63, no. 4, 01 Août 1998, pages 215 - 223, XP001170348
- GERMAIN L. ET AL: 'Biliary epithelial and hepatocytic cell lineage relationships in embryonic rat liver as determined by the differential expression of cytokeratins, [alpha]-fetoprotein, albumin, and cell surface-exposed components' CANCER RESEARCH vol. 48, no. 17, 1988, pages 4909 - 4918, XP008052950
- CHAYA D. ET AL: 'Liver-enriched transcription factors uncoupled from expression of hepatic functions in hepatoma cell lines' MOLECULAR AND CELLULAR BIOLOGY vol. 17, no. 11, 1997, pages 6311 - 6320, XP001091190

## Description

L'invention se rapporte à de nouvelles lignées d'hépatomes humains. Elle vise également leurs obtentions par sélection cellulaire à l'aide des corticostéroïds et DMSO, et leurs applications en diagnostic, thérapeutique et prophylaxie.

L'hépatite B est une maladie infectieuse largement répandue dans le monde. Son virus (VHB en abrégé) est un petit virus à ADN possédant une haute spécificité d'hôte. En effet, seuls l'homme et les primates supérieurs sont infectés par ce virus, ce qui limite fortement les modèles d'études *in vivo* de cette infection. Il existe cependant un modèle dit "du canard" qui permet l'étude d'un cycle entier de réplication d'un hépadnavirus (*in vitro* et *in vivo*). Cet hépadnavirus, bien que proche du virus VHB, présente cependant une biologie assez différente. Par ailleurs, les comportements métaboliques du canard ne peuvent être assimilés simplement à ceux de l'homme.

Le VHB montre de plus un fort tropisme pour le foie et cible préférentiellement les cellules parenchymateuses. Aussi, à la différence avec d'autres virus, seules les cellules hépatiques différenciées sont susceptibles d'être infectées.

Une lignée cellulaire dérivée d'hépatomes a permis de rapides progrès dans la compréhension des mécanismes de réplication. Cette lignée, largement utilisée est référencée sous l'appellation HepG2. Un clone dérivé HepG2 2.2.15 présente l'avantage de posséder une grande capacité de prolifération et de répliquer activement le virus. Cependant, cette réplication n'est possible qu'après l'introduction de l'ADN viral dans les cellules, par transfection. L'infection naturelle de cette lignée par le VHB s'est avérée impossible. De plus, du fait de l'ancienneté de cette lignée, le caryotype de ses cellules est largement modifié, ce qui en fait un modèle imparfait pour mimer les hépatocytes humains. Enfin, la capacité de différenciation de ces cellules reste assez limitée.

D'autres lignées cellulaires plus ou moins différenciées ont été proposées. On citera en particulier la lignée Hep3B, peu différente de la lignée HepG2, et qui présente les même limites que celle-ci. Ces deux lignées font en particulier l'objet du brevet US 4393133, et des articles par Neurath et al. J. exper. med., n° 175, 1992, pages 461-469 et Chiu et al., Hepathology, volume 11, n° 5, 1990, pages 834-842. Tous les essais d'infection de lignées se sont avérés négatifs ou décevants en terme d'efficacité.

Pour étudier les mécanismes d'infection par le VHB, un modèle d'hépatocytes humains en culture primaire a été mis au point. Ce modèle présente le grand intérêt de concerner des hépatocytes humains et de permettre l'accès à l'ensemble du cycle viral. Toutefois, sa manipulation est difficile et fastidieuse, et l'obtention des fragments biopsiques, de plus en plus difficile et aléatoire.

Les travaux des inventeurs dans ce domaine les ont conduits à constater que des lignées d'hépatomes humains se multipliant activement tout en étant infectables par des parasites et/ou des virus, tout spécialement le VHB, pouvaient être obtenues en effectuant une sélection cellulaire dans des conditions spécifiques.

L'invention vise donc à fournir de nouvelles lignées d'hépatomes humains infectables par des parasites et/ou des virus, et les cellules ou éléments de ces cellules issus de ces lignées.

Elle vise également un procédé de sélection de telles lignées.

L'invention a également pour but les applications en diagnostic, thérapeutique et prophylaxie de ces lignées.

Selon la présente invention, les lignées cellulaires d'hépatomes humains sont caractérisées en ce qu'elles sont susceptibles d'être infectées naturellement par des parasites et/ou des virus ; lesdits parasites pouvant être hépatotropes ou non, tels que le *Plasmodium ou les parasites du genre leishmania ;* et expriment des récepteurs à la famille des virus des *Flaviviridae* et *Hepadnaviridae,* de préférence le VHB et le VHC.

Ces nouvelles lignées permettent ainsi l'étude complète du cycle des parasites et/ou des virus notamment le cycle viral du VHB, depuis l'étape d'infection naturelle jusqu'à la réplication et/ou la propagation du virus. On observera à cet égard qu'aucune lignée cellulaire d'hépatomes humains n'est à ce jour infectable par le *Plasmodium falciparum*, c'est-à-dire qu'aucune lignée cellulaire d'hépatomes humains n'est susceptible de supporter le développement de formes matures de ce parasite hépatotrope (schizontes). De plus, du fait de sa capacité à proliférer, ces lignées présentent de grandes facilités de manipulation.

Selon un autre aspect de l'invention, ces lignées sont capables d'atteindre un niveau de différenciation poussée.

En particulier, les cellules issues des lignées selon l'invention permettent d'atteindre un stade de différenciation hépatique, c'est-à-dire une morphologie proche de cellules constituantes du foie, telles que les hépatocytes et/ou les cellules biliaires avec en particulier :
- la formation de travées de cellules parenchymateuses,
- la formation de canalicules biliaires fonctionnels, avec au niveau cellulaire, la reconstitution d'un pôle biliaire.

Dans toute la demande, il sera fait référence à des notions typiques de l'organisation structurelle des cellules du foie. Ces notions, en particulier le pôle biliaire, sont explicitées dans le préambule du paragraphe "Résultats" de l'exemple 1 ci-après. Il est surprenant de constater que les cellules issues des lignées selon l'invention sont capables de mimer quasiment à l'identique l'organisation structurelle des cellules du foie et de reproduire ses fonctions : en effet, les canalicules biliaires formés par les cellules des lignées selon l'invention sont fonctionnels, c'est-à-dire capables de jouer leur rôle de détoxication. Cette différenciation fonctionnelle est d'ailleurs très poussée : les cellules des lignées cellulaires d'hépatomes humains selon l'invention peuvent exprimer les fonctions caractéristiques de l'hépatocyte, à savoir :
- la production de protéines plasmatiques, en particulier l'albumine, et la transferrine,
- la fonction de détoxication, en particulier :
   - l'expression de diverses formes de cytochromes P450, telles que CYP2E1, CYP3A et/ou CYP1A, l'expression de diverses formes d'enzymes de phase II de détoxication en particulier la GSTα,
   - la conjugaison de sels biliaires,
   - l'élimination de l'urée.
- la fonction énergétique régulatrice :
   - le stockage du sucre sous forme de glycogène et la production de glucose par glycolyse, en particulier, l'expression de l'aldolase B, et/ou la néoglucogénèse,
   - le métabolisme des lipides.

On notera avec intérêt qu'aucune lignée cellulaire d'hépatomes humains n'était capable de produire des cellules pouvant atteindre un niveau de différenciation tel que ces cellules puissent exprimer la quasi-totalité des fonctions de l'hépatocyte humain normal, en particulier, les fonctions de détoxication de phases I et II (CYP2E1, CYP3A, CYP1A et GSTα).

Selon un aspect inattendu, les cellules issues des lignées selon l'invention possèdent des propriétés de cellules pluripotentes, en particulier des propriétés de cellules souches résidentes et/ou ovales, c'est-à-dire capables de se différencier vers la voie hépatocytaire, biliaire, pancréatique et/ou intestinale.

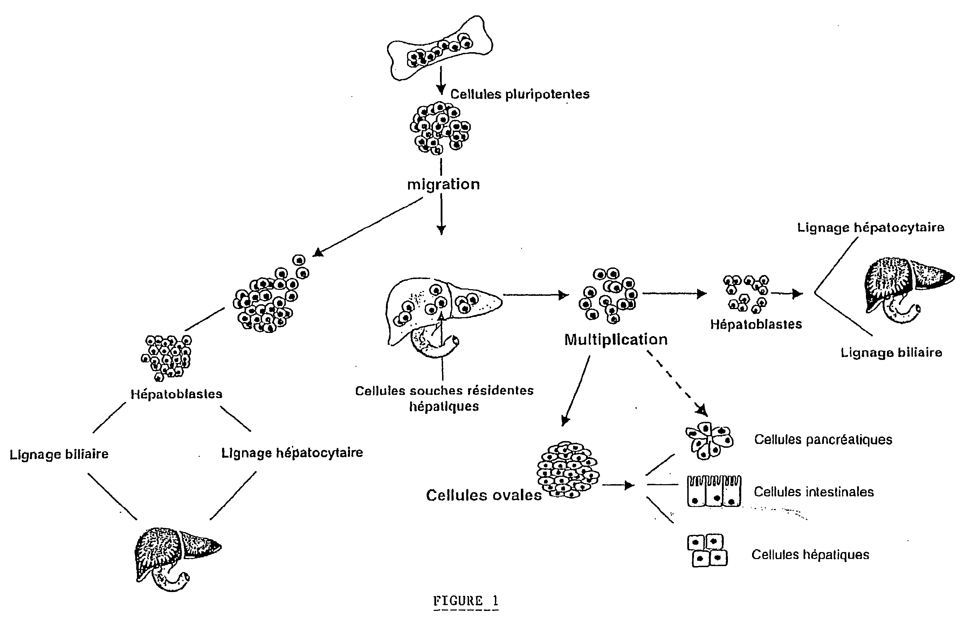

L'article par Zvibel et al., Differentiation, volume 63, n° 4, 1998, pages 215-223, décrit des lignées d'hépatomes de rat présentant des marqueurs biliaires évoluant vers un stade précoce de différentiation hépatocytaire. Ces lignées ne possèdent donc pas la capacité de se différencier par la voie hépatocytaire, biliaire, pancréatique et intestinale. Les cellules souches résidentes sont des cellules du foie, capables, lors d'une destruction massive de celui-ci, de se multiplier activement afin de régénérer la partie détruite. Ces cellules peuvent évoluer vers un lignage hépatocytaire ou biliaire (Figure 1). Par ailleurs, ces cellules souches résidentes parfois aussi désignées en tant que cellules ovales, ont la capacité de se différencier en différents types cellulaires tels que des cellules pancréatiques, intestinales et/ou hépatiques.

Un autre avantage des lignées selon l'invention réside dans leur capacité à proliférer activement. Ainsi, en phase de prolifération, la population cellulaire double en 24h environ.

L'invention vise notamment la lignée cellulaire déposée le 5 avril 2001 à la Collection Nationale de Cultures de Microorganismes, Institut Pasteur, 25 rue du Docteur Roux, F-75724 Paris Cedex 15, sous le n°I-2652.

Cette lignée, appelée HépaRG, est un exemple de lignée cellulaire présentant à la fois toutes les caractéristiques des lignées selon l'invention : elle est infectable naturellement par des parasites, des virus, elle présente les caractéristiques morphologiques énoncées ci-dessus, et possède toutes les fonctions biologiques d'une cellule hépatique. Elle apparaît comme un modèle quasi-parfait des cellules hépatiques.

Par ailleurs, l'invention vise à fournir les méthodes d'obtention et de sélection de lignées hepatiques.

Le procédé de sélection de lignées cellulaires humains comportant :
- une phase de prolifération cellulaire dans un milieu de culture comportant en continu au moins un cortico-stéroïde à une concentration non toxique qui favorise la différenciation des hépatocytes humains normaux, et notamment leur différenciation optimale.
- puis, la confluence atteinte, une phase de différenciation cellulaire dans ce même milieu, par addition de DMSO, en quantité suffisante pour induire la différenciation,
ce procédé étant réitéré plusieurs fois, préférentiellement 3 fois, si souhaité.

L'article par Saito et al., Cancer Biochem. Biophys, volume 15, 1992, pages 75-84 et la demande EP 0 972 828 décrivent des procédés mettant en oeuvre du DMSO ou de la dexaméthasone pour différencier des lignées d'hépatome ou des hépatocytes. Cependant l'utilisation simultanée de DMSO et de corticostéroïde n'est pas proposée.

Ce procédé de sélection cellulaire est indispensable pour le maintien des propriétés des lignées selon l'invention. En effet, ce procédé de sélection induit une mortalité cellulaire élevée. L'exemple 3 ci-dessous démontre la nécessaire coopération entre le cortico-stéroïde et le DMSO.

On appelle "concentration non toxique qui favorise la différenciation des hépatocytes humains normaux", la concentration de cortico-stéroïde favorisant, lors de son ajout à une culture d'hépatocytes humains normaux, la différenciation des cellules vers une morphologique et un état fonctionnel décrit dans le préambule du paragraphe "Résultats" de l'exemple 1. Cette concentration est non toxique, c'est-à-dire que son ajout n'entraîne pas un taux de mortalité cellulaire supérieur à environ 10%.

Par ailleurs, on appelle "quantité suffisante pour induire la différenciation", la quantité de DMSO nécessaire pour induire la différenciation d'une culture d'hépatocytes humains normaux.

On notera avec intérêt qu'un procédé préférentiel comporte un cortico-stéroïde présent à forte concentration, en continu dans le milieu, contrairement aux procédés usuellement utilisés en culture d'hépatomes. De façon surprenante, la présence de ce cortico-stéroïde n'empêche nullement les lignées de proliférer.

Ce procédé de sélection a permis la mise au point d'un procédé d'obtention des lignées selon l'invention comportant une étape de biopsie d'une tumeur solide de type hépatocarcinome, une étape d'isolement de la population cellulaire à l'aide d'une enzyme protéolytique et une étape de sélection des lignées à l'aide du procédé de sélection détaillé ci-avant.

L'enzyme protéolytique préférentiellement utilisée est la trypsine et/ou la collagénase.

L'invention propose également un procédé d'infection de cellules hépatiques par un parasite hépatotrope et/ou un virus comportant :
- une phase de sélection à l'aide du procédé de sélection sus-mentionné,
- une phase de différenciation permettant aux cellules d'atteindre une morphologie proche de l'hépatocyte à l'aide du milieu de culture comportant au moins un cortico-stéroide à une concentration non toxique qui favorise la différenciation optimale des hépatocytes humains normaux, additionné de DMSO, en quantité suffisante pour induire une différenciation,
- une phase d'infection avec incubation des cellules hépatiques dans un milieu de culture comportant au moins un cortico-stéroïde à une concentration non toxique qui favorise la différenciation optimale des hépatocytes humains normaux, additionné de la source infectieuse.

Le parasite hépatotrope peut être un *Plasmodium* et le virus, le VHB ou le VHC.

Une source infectieuse possible est le surnageant de cellules HepG2 et/ou le sérum de malade.

Les milieux de culture utilisés pour les procédés de sélection, d'obtention et d'infection contiennent de préférence de l'insuline en quantité suffisante pour favoriser la survie des hépatocytes humains normaux, préférentiellement de 2,5 µg/ml à 10 µg/ml, en particulier de l'ordre de 5µg/ml.

L'insuline permet d'améliorer considérablement la viabilité des cellules.

Par ailleurs, les cortico-stéroïdes de ces milieux de culture sont de préférence l'hémisuccinate d'hydrocortisone et la dexaméthasone. D'autres inducteurs de différenciation peuvent être utilisés, notamment l'acide rétinoïque et/ou ses analogues de synthèse, les oestrogènes et les hormones thyroïdiennes.

On appelle "analogues de synthèse", les analogues desdits cortico-stéroïdes et rétinoïdes d'origine non naturelle.

La concentration en corticoïde est de 10⁻⁷ M à 10⁻¹ M, préférentiellement de 5.10⁻⁵ M environ pour l'hémisuccinate d'hydrocortisone et de 10⁻⁶ M environ pour la dexaméthasone. Enfin, la concentration en DMSO, lorsque celui-ci est additionné aux milieux de culture, est de 1% à 4%, préférentiellement de 2% environ.

D'autre part, l'invention vise un procédé de transfection des lignées selon l'invention à l'aide d'un vecteur comportant la séquence complète et/ou une partie de matériel génétique des virus VHB et/ou VHC. Elle vise également un procédé de criblage à haut débit de gènes différentiellement exprimés à l'aide d'un outil de type puce réalisé à partir des lignées et/ou des cellules et/ou des parties de cellules selon l'invention, de préférence, ces gènes étant différentiellement exprimés sous l'effet de conditions de culture, de l'exposition à une molécule et/ou un virus et/ou un parasite.

Un exemple d'outil de type puce est donné dans l'exemple 7 ci-après. Il s'agit d'une puce d'ADNc dont la construction permet le criblage à haut débit de gènes différentiellement exprimés.

L'invention couvre enfin l'utilisation des différents milieux de culture permettant l'obtention, l'entretien, la prolifération, la sélection, la différenciation, la transfection, l'infection des lignées selon l'invention. Il s'agit en particulier de l'utilisation d'un milieu de culture comportant en continu au moins un cortico-stéroide, préférentiellement l'hémisuccinate d'hydrocortisone à une concentration non toxique qui favorise la différenciation optimale des hépatocytes humains normaux, afin de maintenir la stabilité de la population cellulaire des lignées et/ou des cellules selon l'invention ; ainsi que l'utilisation d'un milieu de culture comportant en continu au moins un cortico-stéroïde, préférentiellement l'hémisuccinate d'hydrocortisone, à une concentration non toxique qui favorise la différenciation optimale des hépatocytes humains normaux, additionné de DMSO en quantité suffisante afin d'induire une différenciation des cellules issues des lignées selon l'invention ; et de l'utilisation du milieu de culture comportant en continu au moins un cortico-stéroide à la concentration précitée ainsi que du butyrate de sodium à une concentration suffisante pour induire une différenciation de type biliaire, de préférence une concentration de 2,5 à 5 mM, en particulier de 3,75 nM environ.

Comme déjà indiqué, les lignées selon l'invention sont capables d'évoluer vers des voies de différenciation distinctes. La voie de différenciation est fortement influencée par le choix du milieu de culture. Les évolutions vers les voies hépatique, biliaire et pancréatique sont illustrées par l'exemple 4.

La lignée d'hépatomes humains selon l'invention fait l'objet de nombreuses applications étant donné leur capacité de haute différenciation, leur grande fonctionnalité et leur facilité de manipulation.

Une première application intéressante correspond à l'utilisation des lignées et/ou des cellules issues des lignées selon l'invention, pour des tests métaboliques et/ou de toxicité destinés de préférence à l'évaluation de nouveaux médicaments et/ou de constituants nutritionnels et/ou de polluants de l'environnement.

En effet, les lignées selon l'invention sont à ce jour le meilleur modèle mimant les hépatocytes humains normaux, notamment en terme de détoxication.

En particulier, les lignées selon l'invention permettent la fabrication d'un bioréacteur extracorporel pour traiter transitoirement les insuffisances hépatocellulaires aigües. L'invention couvre également l'utilisation des lignées selon l'invention pour le criblage et/ou la fabrication de nouveaux vaccins et/ou de molécules antivirales, en particulier pour le criblage de molécules actives vis-à- vis de l'une des étapes du cycle viral ; pour la fabrication d'anticorps dirigés contre un virus appartenant à la famille des *Flaviviridae* et des *Hepadnaviridae*, en particulier dirigés contre le VHB et VHC et/ou leurs récepteurs membranaires cellulaires ; pour la réalisation de tests de neutralisation virale et/ou de composition vaccinale comprenant au moins des particules virales et/ou des polypeptides obtenus après infection et/ou transfection des lignées selon l'invention, associés à un véhicule et/ou un excipient et/ou un adjuvant pharmaceutique acceptable.

D'autres caractéristiques et avantages de l'invention sont donnés dans les exemples qui suivent. Ils se rapportent, à des fins d'illustration à la lignée I-2652 déposée à la CNCM, appelée HepaRG. Dans ces exemples, il est fait référence aux figures 1 à 13, qui représentent respectivement :
- la figure 1, une représentation schématique des différents types cellulaires participant à l'homéostasie du foie,
- la figure 2, une coupe schématique du foie explicitant l'organisation structurelle des différentes cellules constitutives du foie,
- la figure 3, la courbe de croissance de la lignée HepaRG,
- la figure 4, des microphotographies en contraste de phase des cellules HepaRG à différents stades de différenciation,
- la figure 5, des micrographies électroniques des cellules HepaRG,
- la figure 6, le caryotype à coloration de type RHG d'une métaphase pseudodiploïde représentative de la lignée HepaRG,
- la figure 7, une analyse par Northern blot de l'expression des ARNm dans 2 biopsies de foie, les cellules HepG2 et les cellules HepaRG,
- la figure 8, les effets d'inducteurs des activités CYP1A (phénacetine dééthylase) et CYP3A4 (nifédipine oxidase),
- la figure 9, l'influence des corticoïdes et du DMSO sur le niveau des ARNm hépatiques dans les cellules HepaRG,
- la figure 10, les effets de différents facteurs influençant l'infectabilité des cellules, et
- la figure 11, les résultats de l'infection des cellules HepaRG par le VHB.
- les figures 12 et 13, les différenciations biliaires et pancréatiques,
- les figures 14 et 15, l'infectabilité des cellules HépaRG par le sérum de patients porteurs du VHC, en présence ou en l'absence d'interféron α,
- la figure 16, la cinétique de réplication ou d'inhibition du VHC par l'interféron α,
- la figure 17, les cinétiques d'infection par des parasites du genre *Leishmania,* et,
- les figures 18 et 19, des optimisations du protocole d'infection par les parasites du genre *Leishmania.*

### Exemple 1 : Isolement de lignée cellulaire d'hépatomes

### 1. Matériels et méthodes

L'isolement des cellules est effectué à partir d'une tumeur de foie prélevée sur une patiente souffrant d'un hépatocarcinome et d'une hépatite virale C. Toute la procédure a été menée en conformité avec la loi et les règlements français et approuvée par le Comité National d'Ethique, et de manière confidentielle.

Les prélèvements sont découpés en fines lamelles, puis rincés avec une solution tampon à base d'HEPES [(pH 7.7) ; 140 mM NaCl, 2.68 mM KCl, 0.2 mM Na₂HPO₄ et 10 mM HEPES], et digérés avec 0.025% de collagénase D (Boehringer Mannheim) diluée dans la même solution tampon additionnée de 0.075% de CaCl₂ (addition sous agitation douce à 37°C). Après deux lavages avec la solution tampon HEPES, les cellules sont remises en suspension dans un milieu William's E, supplémenté avec 10% de sérum de veau foetal (SVF), 100 U/ml de pénicilline, 100 µg/ml de streptomycine, 5 µg/ml d'insuline, 2 mM/ml de L-glutamine et 5.10⁻⁷M d'hémisuccinate d'hydrocortisone. La suspension cellulaire est ensuite répartie dans différents puits sur support plastique. Après plusieurs semaines, la croissance cellulaire est suffisante pour pouvoir réaliser une culture. Sa population est hétérogène, mais des cellules sont très différenciées et présentent une morphologie de type hépatocyte. Les puits présentant les populations cellulaires les plus homogènes sont décollés à la trypsine, puis redistribués. Après 3 passages, les cellules sont aliquotées, puis congelées dans le milieu de culture additionné de 10% de DMSO et conservées dans l'azote liquide. Après décongélation, le puits présentant la plus grande proportion de cellules ayant une morphologie de type hépatocytaire est sélectionné. Les cellules sont cultivées dans les milieux de culture utilisés pour leur isolement et/ou dans le milieu de différenciation utilisé pour parfaire la sélection cellulaire.

Pour obtenir une différenciation hépatocytaire plus fréquente au sein de la lignée, les cellules HepaRG issues de la première sélection sont cultivées dans un milieu de William's E, additionné de 5 µg/ml d'insuline, 100 U/ml de pénicilline, 100µg/ml de streptomycine, 5.10⁻¹ M d'hémisuccinate d'hydrocortisone, 2 mM de L-glutamine et 10% de SVF.

Les cellules sont soumises à un passage tous les 10-15 jours, à une dilution de 1/5. La phase de différenciation s'effectue en deux temps :
- les cellules sont maintenues dans leur milieu de croissance pendant deux semaines, la confluence étant atteinte au bout d'une semaine,
- elles sont ensuite maintenues dans un milieu de différenciation (correspondant au milieu de culture précédent, additionné de 2% de DMSO) pendant deux autres semaines, le milieu étant renouvelé tous les 2 ou 3 jours.

### Analyse cytogénétique :

Le caryotype des cellules HepaRG a été analysé après 8 passages. Les cellules ont d'abord été entretenues pendant 24 à 48 heures dans du RPMI 1640 additionné de 10% de SVF, puis bloquées en métaphase par une exposition à la Colcémide (10 µg/mL) pendant 45 minutes.

Les cellules ont ensuite été traitées avec une solution hypotonique (0,1 M de MgCl₂), fixées avec une solution acétique de Carnoy et les chromosomes révélés par coloration RHG.

### 2.Résultats

### Préambule

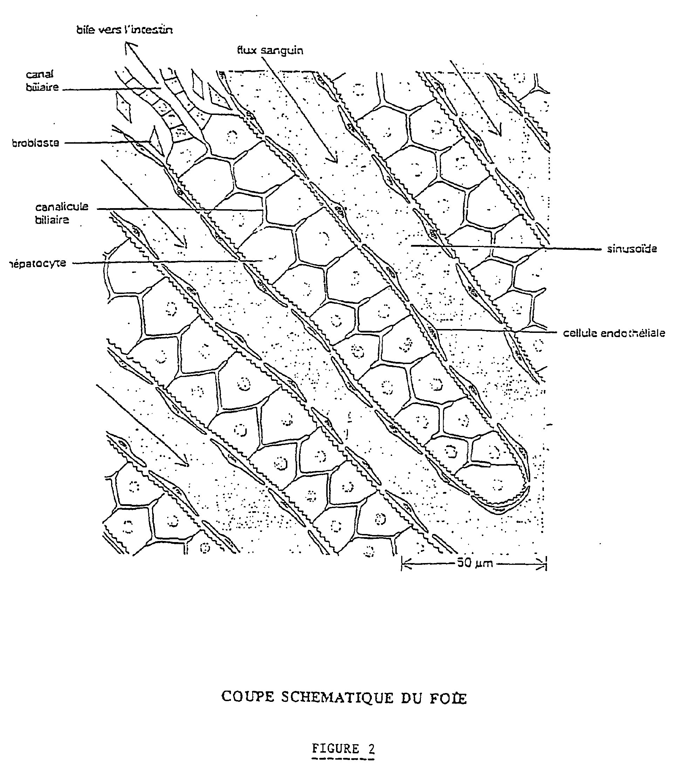

La figure 2 donne une coupe schématique du foie explicitant l'organisation structurelle des cellules du foie. En particulier, un hépatocyte humain normal présente les caractéristiques morphologiques suivantes :
- Ce sont des cellules avec un cytoplasme granuleux du fait de la richesse en organites tels que les mitochondries et les vésicules de réticulum endoplasmique rugueux, et un noyau central rond et régulier avec un nucléole très dense.
- Ces cellules se regroupent et s'organisent en travées typiques, le plus souvent formées de cordes de 2 à 4 cellules, liées entre elles par des structures jonctionnelles de type desmosome, et des structures communicantes de type "Gap" et bordées de chaque côté par des sinusoïdes dans lesquels circule le sang. Cette organisation en travées détermine la double polarité qui caractérise l'hépatocyte : un pôle sinusoïdal du côté des sinusoïdes et un pôle biliaire à l'interface des hépatocytes.

Le pôle biliaire est associé à la fonction de détoxication et plus particulièrement à l'élimination des sels biliaires. Il s'agit d'un espace intercellulaire dilaté, fermé par des jonctions complexes caractéristiques (jonctions serrées plus desmosomes) qui délimitent pour chaque cellule une zone membranaire spécialisée d'une part, au plan fonctionnel par l'expression de protéines spécifiques, et d'autre part, au plan morphologique par la formation de nombreuses villosités.

De plus, on appelle cellules biliaires, les cellules constitutives des canaux et canalicules biliaires. Ceux-ci permettent d'évacuer vers la bile, les sels biliaires provenant des hépatocytes et ayant circulé le long de la travée de cellules parenchymateuses par l'intermédiaire du pôle biliaire.

### Sélection des cellules présentant une morphologie d'hépatocyte

Les cellules issues des puits initialement sélectionnés pour leur forte proportion en cellules de type hépatocytaire sont entretenues dans un milieu de culture contenant 5.10⁻⁷M d'hémisuccinate d'hydrocortisone. On constate que leur morphologie devient de plus en plus hétérogène et éloignée de celle d'un hépatocyte.

Afin de retrouver une population cellulaire de type hépatocytaire, on utilise alors conformément à l'invention, la méthode de sélection suivante :
- on utilise un milieu de différenciation formé à partir d'un milieu de base contenant seulement 5 mg/L d'insuline et 10% de SVF auquel on ajoute 2% de DMSO et 5.10⁻⁵M d'hémisuccinate d'hydrocortisone. Une fois la confluence bien établie, les cellules HepaRG sont cultivées dans ce milieu.

Dans les 2 à 4 jours suivants, le milieu, très toxique, provoque la mort de plus de 90% des cellules. Parallèlement, la morphologie de certaines cellules survivantes change considérablement.

Après deux semaines de ce traitement, le taux de mortalité des cellules apparaît nul et certaines cellules survivantes présentent la même morphologie que les hépatocytes humains normaux, cultivés dans les mêmes conditions.

On constate donc que les cellules différenciées sont plus résistantes au DMSO que les autres.

Les cellules, une fois replacées dans le milieu dépourvu de DMSO, retournent vers une phase de prolifération active.

Le procédé de sélection est poursuivi. Après 3 étapes de sélection dans ce milieu de différenciation, la plupart des cellules deviennent résistantes au DMSO et capables de se différencier à nouveau, une fois la confluence atteinte.

L'homogénéité de la population est encore améliorée grâce à deux autres protocoles de sélection.

Nous avons observé et mis à profit le fait qu'un traitement à la trypsine tend à décoller les cellules les plus différenciées sous forme d'amats multi-cellulaires tandis que les cellules les moins différenciées sont isolées. La purification des gros agrégats permet d'enrichir la population en cellules susceptibles de se différencier.

L'utilisation de la collagénase est basée sur un principe très différent : le traitement ménagé à la collagénase entraîne le décollement sélectif des cellules les plus différenciées qui peuvent être récupérées et réensemencées.

Enfin, la stabilité du phénotype de la lignée est grandement favorisée par l'utilisation en continu d'une forte concentration d'hémisuccinate d'hydrocortisone (5.10⁻⁵M).

### Les modifications morphologiques de la prolifération à la différenciation

Une caractéristique inattendue des cellules sélectionnées concerne leur aptitude à proliférer en présence de fortes concentrations en corticoïde. Après un décollement, la plupart des cellules attachent au support en deux heures et se remettent à proliférer. Durant la phase exponentielle de prolifération, la population double en 24 heures puis, lorsque les cellules arrivent à confluence, la croissance ralentit. Celle-ci atteint un plateau dans les 10 jours suivant le passage, après quoi, les cellules ne peuvent plus se diviser, mais restent vivantes pendant plusieurs semaines.

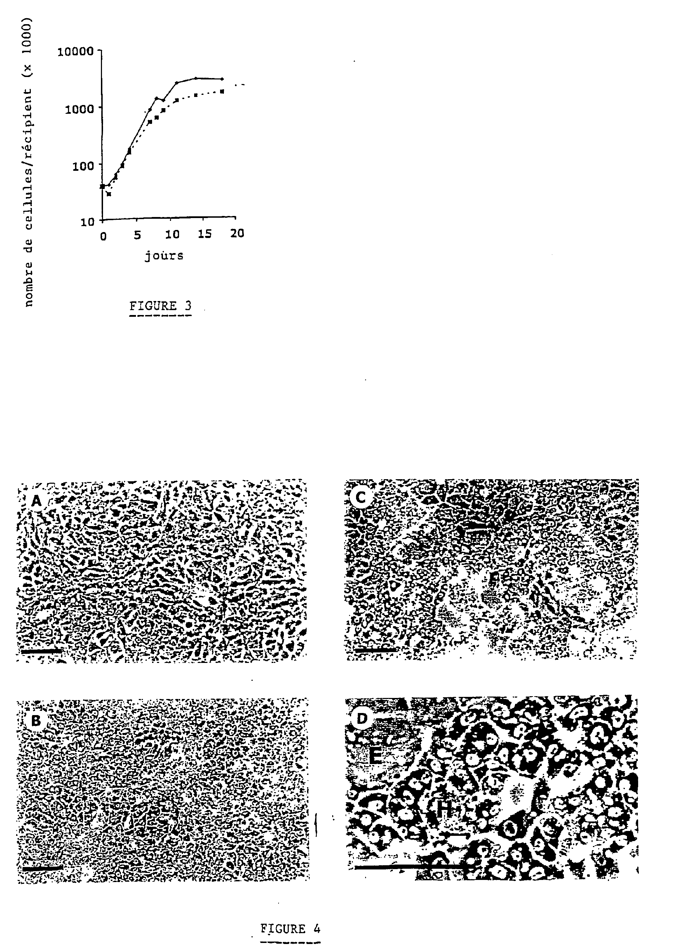

Sur la figure 3, les cellules cultivées en l'absence - ■ - ou en présence d'hydrocortisone (5.10⁻⁵ M) -◆- pendant 10 passages ont été ensemencées à la densité de 40 000 cellules par puits de 4 cm² et maintenues dans le même milieu de culture. Les cellules ont été collectées par trypsination et comptées manuellement.

Durant la phase de prolifération, les cellules HepaRG forment une population homogène de phénotype épithélial (absence d'organisation structurelle régulière) et de formes différentes de celle des hépatocytes (Fig. 4A, cellules HepaRG dans des conditions de prolifération). Une fois la confluence atteinte, les cellules subissent des modifications morphologiques importantes. En quatre semaines, de nombreuses colonies de cellules granuleuses de type hépatocytaire apparaissent, tandis qu'à la périphérie, on peut voir distinctement des cellules épithéliales (Fig. 4B, cellules HepaRG maintenues à confluence pendant 20 jours). L'addition de DMSO deux semaines après le passage induit une différenciation morphologique très complète : organisation des cellules en travées comparables à celles obtenues en culture primaire d'hépatocytes normaux, dans lesquelles on peut reconnaître des structures de type canaliculaires (indiqué par une flèche noire sur la figure 4D). Les figures 4C et 4D présentent des cellules HepaRG maintenues à confluence pendant 5 jours, puis traitées avec 2% de DMSO pendant 15 jours (Echelle = 100 µm).

Quelques cellules épithéliales occupent les espaces libres (cellules plates, régulières, en clair sur les figures 4C et annotées par la lettre E sur la figure 4D). La morphologie caractéristique hépatocytaire se traduit aussi au niveau de l'organisation en travées des cordes d'hépatocytes. Elle est obtenue au bout de deux semaines d'exposition au DMSO, les cellules granuleuses ressemblant alors très fortement à des hépatocytes (Fig. 4C, cellules annotées par la lettre H sur la figure 4D). Au bout de ces deux semaines, on ne constate plus de modifications significatives.

Les conditions permettant d'obtenir une différenciation hépatocytaire complètes sont donc définies par :
- une semaine de confluence en présence de corticoïde (5.10⁻⁵M d'hydrocortisone),
- puis deux semaines de différenciation en présence d'hydrocortisone et de 2% de DMSO.

De façon surprenante, il a été observé qu'en collectant sélectivement la population cellulaire granuleuse des travées, celle-ci était capable, même après une longue période de quiescence, de retourner en phase de prolifération active et de donner naissance à nouveau aux deux types cellulaires hépatocytaire et épithélial lorsque la confluence est atteinte.

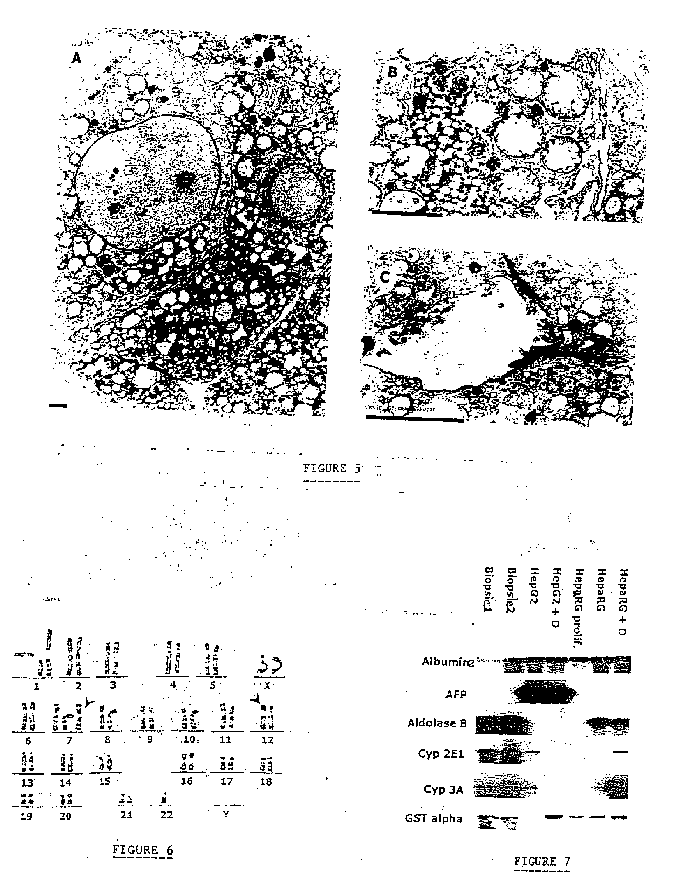

Une étude au microscope électronique a permis de mettre en évidence l'organisation structurelle des cellules : deux cellules voisines sont fortement attachées entre elles par des desmosomes (Fig. 5A, vue à faible grossissement), avec des structures ressemblant beaucoup à des canalicules biliaires et entourées par des complexes typiques de jonctions (jonctions "serrées" + desmosomes). Les figures 5B et 5C présentent des vues à plus fort grossissement, montrant une accumulation typique de granules de collagène et une structure de type canalicule biliaire. (Echelle = 2 µm).

Sur la figure 5A, on peut voir des noyaux ronds réguliers, caractéristiques, comportant un ou deux nucléoles. Les très nombreuses mitochondries du cytoplasme ont une forme légèrement bombée présentant quelques pics. Il est aussi possible d'observer de nombreux granules cytoplasmiques, qui sont en fait une accumulation de particules de glycogène organisées en "rosette" tel que décrit dans le foie normal *in vivo* (Fig. 5B).

Enfin, de manière inattendue le caryotype des cellules de la lignée est subdiploïde. La formule caryotypique suivante a été déduite : 46 <2n>, XX, + del (7) (q11 q21) inv? (7) (q21 q36), -der (22) t(12;22) (p11 ; q11). La détermination des points de délétion et de translocation a été réalisée par hybridation *in situ*. Sur 40 mitoses étudiées, 65% contiennent 46 chromosomes. 100% des cellules présentent les anomalies suivantes (Fig. 6) _{:}
- un chromosome 7 surnuméraire et modifié conduisant à une trisomie 7, et
- une translocation t (12,22) avec une perte du fragment 12p conduisant à une monosomie 12p (Fig. 6).

D'autres anomalies ont été ponctuellement détectées et sont rapportées dans le Tableau I suivant :

**TABLEAU I : CARACTERISTIQUES CYTOGENETIQUES DE LA LIGNEE HEPARG**

| **CHROMOSOMES AVEC ANOMALIES** | | | |
|---|---|---|---|
| **CHROMOSOME** | **ANOMALIES** | | **FREQUENCE** **(% DE MITOSES)** |
| 7 | ? inv (7) (q21q36) del(q11-q21) surnuméraire | Trisomie partielle | 100 |
| 12 | Translocation déséquilibrée | Monosomie 12p | 100 |
| 22 | t(12;22)(p11;q11) | Monosomie 22p | 100 |
| 2 | surnuméraire | Trisomie | 15 |
| 4 | surnuméraire | Trisomie | 7.5 |
| 8 | i(8)(q10) | Monosomie 8p | |
| | | Monosomie 8q | 5 |

### Exemple 2 : Capacité fonctionnelle de la lignée

### 1.Matériels et méthodes

### Extraction de l'ARN et analyses

L'ARN cellulaire total est extrait à l'aide du kit Total SV RNA^{®} (Promega, France), séparé sur gel d'agarose 1.5% et analysé par Northern blot. Le contrôle de la quantité d'ARN transféré sur les filtres est effectué après un marquage au bleu de méthylène. L'hybridation est effectuée selon le protocole de Gripon et al. (1993, Virology, 192:534-540).

### Activités enzymatigues associées à la fonction de détoxication

Les activités enzymatiques sont établies grâce aux protocoles mis au point par Guillouzo et al. (1993, Hepatology 18:406-414**)***.*

Ont été étudiés :
- la dééthylation de la phénacétine en paracétamol,
- l'oxydation de la nifédipine en 3,5-diméthoxy-carbonyl-2,6-diméthyl-4-(2-nitro-phényl) pyridine,
- la déméthylation du dextrométhorphane en tartrate de dextrorphane,
- l'hydroxylation du tolbutamide en hydroxytolbutamide,
- l'hydroxylation de la 4-méphénytoïne en 4-hydroxy-méphénytoine.

Pour comparaison, les activités enzymatiques ont aussi été déterminées sur des cultures d'hépatocytes humains normaux incubées pendant 2 à 16 heures en présence des substrats suivants :
- 2.10⁻⁴ mol/L de phénacétine
- 2.10⁻⁴ mol/L de nifédipine
- 2.10⁻⁴ mol/L de dextrométhorphane et de méphénytoïne
- 1 mmol/L de tolbutamide.

Les dosages sont effectués par chromatographie liquide haute performance (HPLC). Les résultats sont exprimés en picomoles de métabolites formés par heure et par µg d'ADN. L'activité de la glutathion-S-transférase (GST) est estimée en utilisant le 1-chloro-2,4-dinitro-benzène (CDNB) (Merck) comme substrat, à pH 6.5 et à température ambiante.

### 2.Résultats

Le niveau de différenciation des cellules a été contrôlé par analyse des quantités de différents ARNm spécifiques du foie, en particulier les ARNm des protéines des fonctions spécifiques du foie, comme les protéines du sérum (albumine, transferrine), une enzyme hépatique impliquée dans la glycolyse (aldolase B) et 3 enzymes spécifiques impliquées dans la détoxication (CYP2E1, CYP3A et GSTα). Tous ces ARNs sont exprimés par les hépatocytes adultes.

Leurs niveaux d'expression en phase de prolifération et de différenciation ont été comparés. De plus, une comparaison a été établie avec les niveaux d'expression correspondant chez les hépatocytes humains et les cellules HepG2.

La figure 7 est une analyse par Northern blot de l'expression des ARN messagers dans 2 biopsies de foie, les cellules HepG2 et les cellules HepaRG. Les cellules ont été maintenues soit en conditions de prolifération (prolif) soit à confluence pendant 1 mois. Un traitement des cellules avec 2% de DMSO pendant les 15 derniers jours de culture est indiqué comme suit : +D.

On détecte peu ou pas d'ARNm hépatiques spécifiques dans les cellules en prolifération. En revanche, on peut en détecter dans les cellules à confluence, maintenues pendant 2 semaines en présence d'une forte concentration de corticoïde. Ceux de l'albumine et de l'aldolase B sont fortement exprimés, tandis que CYP2E1 et CYP3A4 le restent faiblement. On observe également que dans les cellules ayant acquis l'organisation typique en travées, l'exposition au DMSO provoque une augmentation des transcrits correspondant à l'expression accrue de ces deux dernières enzymes, rendant leur niveau d'expression pratiquement égal à celui observé dans les cellules issues de biopsies.

L'étude menée en parallèle sur les cellules de la lignée HepG2 utilisée en référence révèle que 3 des 5 fonctions spécifiques étudiées sont peu ou pas exprimées chez ces cellules. De plus, l'action du DMSO sur les HepG2 semble inhiber l'expression de certaines de ces fonctions, en particulier CYP2E1 et CYP3A.

Ces analyses mettent en évidence le caractère original de la lignée HepaRG.

La mesure de l'activité de ces différentes enzymes correspondant aux phases I et II de détoxication a ensuite été effectuée. Les autres lignées cellulaires expriment faiblement ces enzymes, souvent tardivement, ou encore ne répondent pas à des inducteurs spécifiques.

L'activité de ces différentes enzymes a été mesurée sur des cellules HepaRG confluentes traitées au DMSO. Cette activité a été comparée à celle de cultures primaires d'hépatocytes humains, ainsi qu'à celle des lignées HepG2 et le clone BC2 de la lignée HBG. Les résultats sont donnés dans le Tableau II suivant :

**TABLEAU II : ACTIVITE DES ENZYMES DE PHASE I ET II**

| **ACTIVITE ENZYMATIQUE** | **HepaRG** | **LIGNEE D'HEPATOMES HUMAINS** | | **CULTURE PRIMAIRE D' HEPATOCYTES HUMAINS** | | |
|---|---|---|---|---|---|---|
| | | **HBG BC2** | **HEPG2** | **Min/Max** | **Moyenne** | **n** |
| Phénacétine dééthylase* | 0.33 | 0.1 | 0.3 | 0.1-25 | 3.9 | 47 |
| Tolbutamide hydroxylase | 0.51 | 0.03 | <0.2 | 0.2-2.1 | 0.9 | 8 |
| S-méphénytoine hydroxylase * | 0.45 | <0.1 | ND | 0.1-2 | 0.7 | 10 |
| Dextrométhorphane déméthylase * | 0.06 | 0.02 | <0.1 | 0.1-2 | 0.5 | 10 |
| Nifédipine oxydase * | 1 | 1 | <0.5 | 0.5-30 | 5.7 | 34 |
| Paracétamol glucuronyle transférase* | 3.7 | 1.5 | <0.3 | 0.3-16 | 4.1 | 26 |
| Paracétamol sulfoconjugation* | 0.7 | 0.16 | 0.3 | 0.1-14 | 3.6 | 27 |
| Glutathione S-transférase* (substrat : chrorodinitrobenzène) | 0.04 | 0.008 | ND | 0.03-0.5 | 0.12 | 27 |

| | | | | | | |
|---|---|---|---|---|---|---|
| * *activité exprimée en nanomoles de métabolites produits*/*h*/*mg de protéines* + *activité exprimée en unités*/*mg de protéines* | | | | | | |

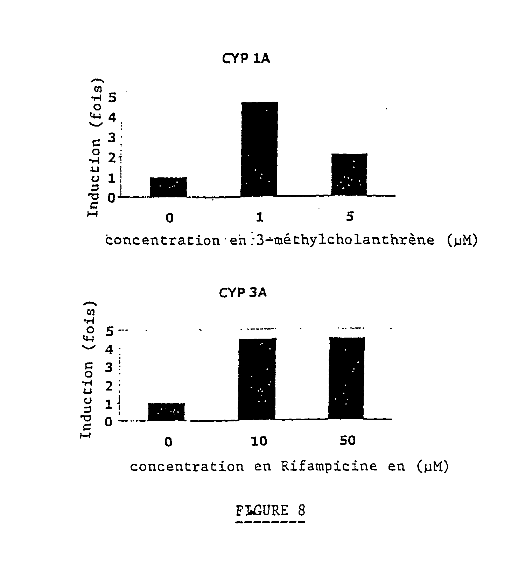

L'examen de ce tableau montre que les cellules HepaRG possèdent les activités enzymatiques associées à la fonction de détoxication à des taux quasi équivalents à ceux exprimés par les hépatocytes humains normaux, excepté pour la dextrométhorphane déméthylase dont l'activité est légèrement inférieure. L'activation de la phénacétine dééthylase et de la nifédipine oxydase est importante comparée à celle obtenue avec les autres lignées. Cette activation est inférieure à celle observée dans les hépatocytes humains normaux, tout en restant dans le même ordre de grandeur (Fig. 8, les activités ont été mesurées après 72h de traitement et sont exprimées en rapport des cellules traitées versus les cellules non traitées correspondant aux cellules contrôles).

### Exemple 3 : Coopération nécessaire entre le corticoïde et le DMSO pour une différenciation complète

Des expériences ont été menées afin de vérifier si les deux agents étaient nécessaires pour induire une différenciation complète.

### 1. Effet sur la différenciation

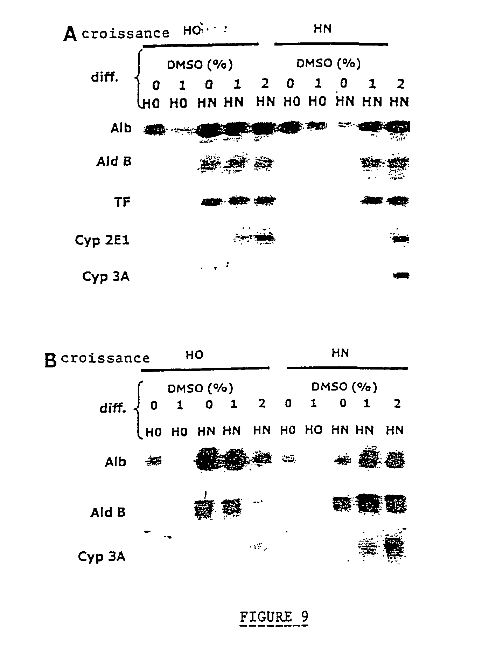

Une première série d'expériences a été réalisée pour étudier les effets de l'absence du corticoïde sur des cellules ayant toujours été entretenues en sa présence. Sur la figure 9A, les cellules HepaRG au passage 13, ont été maintenues pendant 2 passages supplémentaires soit en la présence continue d'hydrocortisone (croissance : HN), soit en l'absence d'hydrocortisone (croissance : HO). Elles ont ensuite été induites à se différencier (diff.) pendant 3 semaines en absence d'hydrocortisone (HO) ou en présence d'hydrocortisone 5x10⁻⁵ M (HN), et en présence de différentes concentrations de DMSO.

Le retrait du corticoïde a été effectué à confluence, c'est-à-dire au moment où les cellules débutent leur différenciation.

Après 2 semaines de culture, en l'absence de DMSO, aucune fonction spécifique (aucun transcrit) du foie, excepté l'albumine, n'est détectée. L'addition de 2% de DMSO est si toxique que son utilisation est impossible. L'addition de 1% de DMSO, moins toxique, n'induit pas de différenciation plus poussée.

Une seconde série d'expériences a permis d'étudier les effets dus à l'absence de DMSO.

Lorsque les cellules sont seulement exposées au corticoïde, la présence de quelques transcrits (de l'albumine, de la transferrine et de l'aldolase B) est détectée à des taux très faibles. L'addition de 1 à 2% de DMSO au milieu de culture provoque une accumulation rapide de nombreux transcrits, en particulier ceux déjà cités et ceux du CYP2E1 et du CYP3A.

Ceci démontre que la coopération entre le corticoïde et le DMSO est indispensable pour atteindre une différenciation maximale. Enfin, la stabilité des cultures a été constatée sur au moins 6 semaines.

### 2. Effet sur la prolifération : utilisation continue du corticoïde

Les cellules sont entretenues dans un milieu de culture privé de corticoïde et leur capacité à mener une différenciation est testée après 2 (Fig. 9A) et 10 passages (Fig. 9B).

Dans les deux cas, aucune différenciation n'est observée et seul l'ARNm de l'albumine est détecté. L'addition de 2% de DMSO se révèle très toxique et l'addition de 1% de DMSO ne provoque pas de changements notables.

L'addition de corticoïde seul à confluence, provoque, en revanche, une accumulation des différents transcrits spécifiques du foie. Mais cette différenciation est incomplète car les transcrits de CYP2E1 et de CYP3A sont difficilement détectés, même après l'ajout de 1 ou 2% de DMSO, ce dernier dosage se révélant très toxique après 10 semaines sans corticoïde.

Enfin, les cellules privées de corticoïde subissent peu à peu des modifications de leur croissance : l'inhibition de contact est retardée si bien que le plateau de confluence présente une densité de cellules deux fois supérieure à celle constatée avec des cellules continuellement entretenues en présence de corticoïde (Fig. 3).

Cette étude montre bien l'importance de la coopération entre le DMSO et le corticoïde tant au niveau de la phase de prolifération (le corticoïde permettant de diminuer la toxicité du DMSO) que de la phase de différenciation (où l'action des deux agents est complémentaire).

### Exemple 4 Capacités des cellules HepaRG à évoluer vers les voies de différenciation biliaire et pancréatique

### 1. Matériels et méthodes

Les cellules HepaRG ont été décollées, diluées au ¹/₅ dans du milieu William's E additionné de 5 µg/ml d'insuline, 100 U/ml de pénicilline, 100 µg/ml de streptomycine, 5 10⁻⁵M d'hémisuccinate d'hydrocortisone, 2mM de L-glutamine et 10% SVF, puis redistribuées dans des puits à support plastique. Quatre conditions de cultures ont été réalisées :
- les cellules HepaRG ont été traitées avec du butyrate de sodium à 3,75 mM dans le milieu d'entretien dès le lendemain du repiquage selon le protocole décrit par Blouin et al. (1995, Specialization switch in differentiating embryonic rat liver progenitor cells in response to sodium butyrate, Exp. cell res., 217:22-33). Le milieu est ensuite renouvelé tous les 2-3 jours,
- les cellules HepaRG ont été traitées 5 jours après repiquage par le butyrate de sodium à 3,75 mM ou le DMSO 2%. Le milieu a été renouvelé tous les jours durant 5 jours,
- les cellules HepaRG ont été traitées à forte confluence par le butyrate de sodium à 3,75 mM ; le milieu a été renouvelé tous les 2-3 jours,
- les cellules HepaRG ont été ensemencées sur support plastique ou sur monocouche de cellules épithéliales primitives biliaires de rat dans du milieu MEM alpha complémenté en L-glutamine (2mM), en i-inositol (0.2mM), en acide folique (20mM), en β-mercaptoéthanol (10⁻⁴ M), en transférine 200µg/ml, en SVF 12.5% et sérum de cheval 12.5%. Dans certaines situations, des cytokines comme le LIF, le IL-3, le SCF ou le G-CSF ont été additionnées au milieu. Ce milieu a été renouvelé tous les 2-3 jours.

### Immunohistochimie indirecte

Les cellules après lavage au PBS sont fixées par une solution de paraformaldéhyde 4% tamponnée par du cacodylate de sodium 0.1 M à pH 7,4 pendant 20 minutes à 4°C. Elles sont ensuite conservées dans un tampon PBS jusqu'au moment de l'analyse.

Les sites spécifiques sont saturés pendant 45 minutes avec du PBS 10% SVF. Les échantillons sont ensuite traités avec les anticorps primaires pendant une heure à température ordinaire dans du PBS 0.05% saponine. Après 3 lavages en PBS 0.05% saponine, les échantillons sont incubés avec le second anticorps couplé à la péroxydase. Deux lavages sont ensuite réalisés, puis l'activité péroxydase est révélée par une incubation avec 0.4mg/ml de 3,3'-diaminobenzidine dans une solution de Tris 0.05 M à pH 7.6, 0.01% H₂O₂ à 110 volumes.

### 2. Résultats

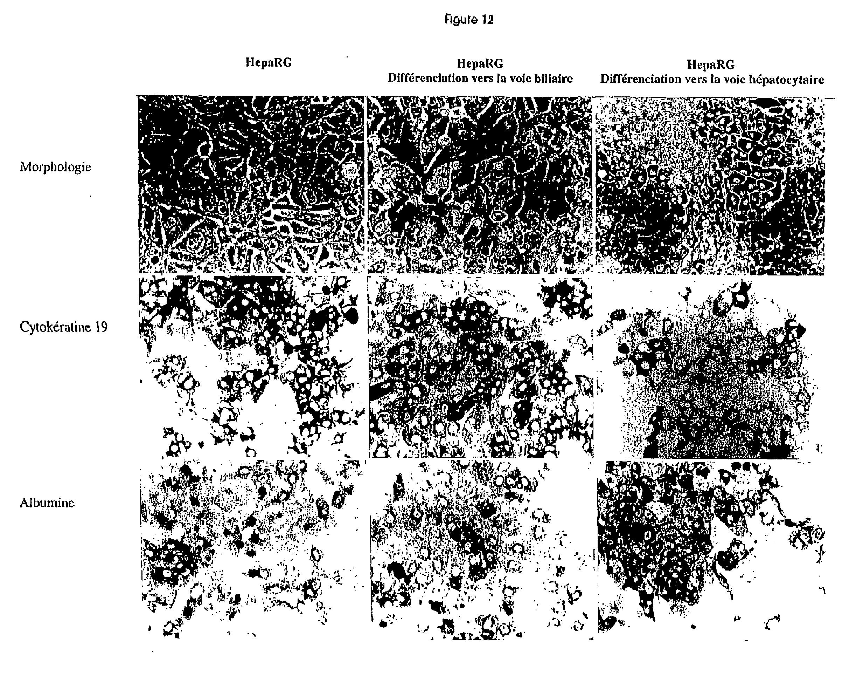

### a/ Différenciation vers la voie biliaire (figure 12)

### Modification morphologique

Avant tout traitement et à confluences, les cellules HepaRG constituent une population peu homogène où l'on trouve des cellules polygonales et des cellules allongées (figure 12).

Dans toutes les conditions de traitement à confluence des cellules HepaRG par le butyrate de sodium, les cellules présentent la morphologie particulière de cellules biliaires en culture (figure 12). Les cellules s'étalent et augmentent de taille, elles présentent un cytoplasme clair avec un noyau ovoïde contenant plusieurs nucléoles. Le contour des cellules est généralement mal défini. Parfois, des gouttelettes lipidiques sont observées. Lorsque les cellules sont traitées à très forte confluence, certaines des cellules meurent. Ces cellules correspondent vraisemblablement aux cellules déjà engagées vers la voie hépatocytaire.

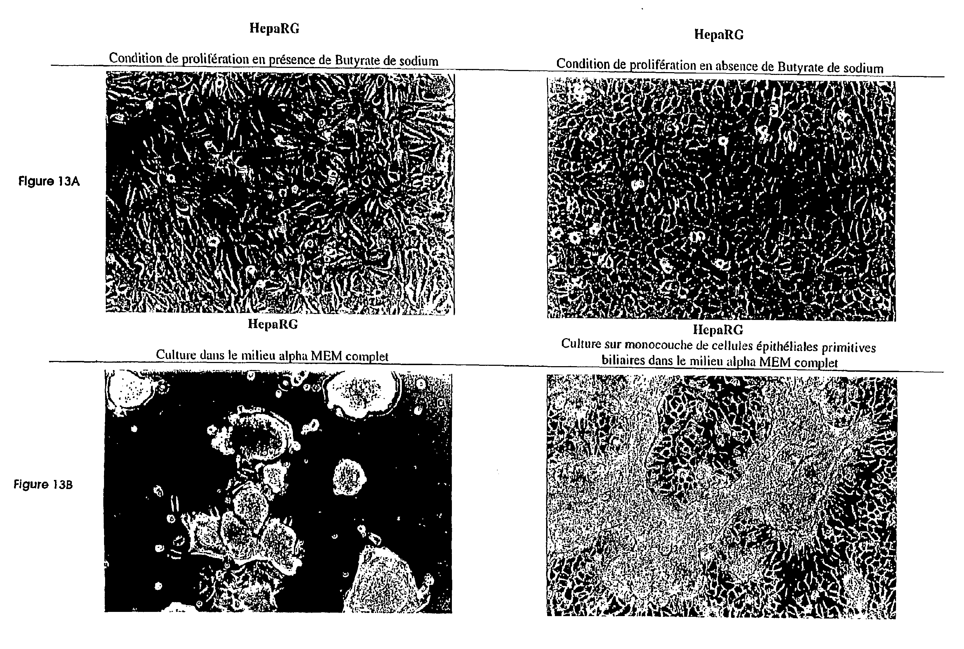

L'effet du butyrate a aussi été testé sur des cellules HepaRG à faible densité et en phase active de prolifération. Une caractéristique de ces cellules est leur aptitude à proliférer en présence de forte dose de butyrate de sodium (figure 13A). Durant la phase de prolifération, les cellules HepaRG forment une population homogène de cellules de type épithélioïde, à confluence elles présentent un aspect de cellules épithéliales biliaires.

### Changements phénotypiques

Les modifications morphologiques observées en présence de butyrate de sodium sont corrélées à des changements phénotypiques (Blouin et al. **1995**)**.** Nous avons utilisé quatre marqueurs de différenciation vers la voie biliaire, la γ-glutamyl transférase, la chaîne α6 des intégrines et les cytokératines 7 et 19 qui normalement disparaissent lorsque les cellules se dirigent vers la voie hépatocytaire. Pour la différenciation vers la voie hépatocytaire, nous avons recherché une augmentation de l'expression de l'albumine et une diminution de l'expression de la chaîne α1 des intégrines.

Dans un premier temps nous avons caractérisé les cellules proliférantes non différenciées. Les cellules de la lignée HepaRG présentent le phénotype suivant : expression du c-kit, des cytokératines 7 et 19, des chaînes α1 et α6 des intégrines, de la γglutamyl transférase et de l'albumine suggérant que ces cellules sont des cellules ovales et/ou des cellules souches (voir schéma figure 1) .

Lorsque les cellules sont cultivées en présence de butyrate de sodium, elles expriment plus fortement la γglutamyl transférase, conservent l'expression de la chaîne α6 des intégrines et des cytokératines 7 et 19 attestant de leur engagement vers la voie biliaire. Lorsqu'elles sont cultivées en présence de DMSO, inducteur de différenciation hépatocytaire, nous observons une disparation de l'expression des cytokératines 7 et 19, de la chaîne α6 des intégrines. Elles expriment à un moindre niveau la chaîne α1 des intégrines et très fortement l'albumine confirmant leur différenciation hépatocytaire. Les populations cellulaires étant hétérogènes dans les cultures, ces phénotypes très caractéristiques ne sont retrouvés que dans les plages se différenciant vers l'une ou l'autre des voies.

Les résultats obtenus après marquage *in situ* des cellules traitées ou non par le butyrate de sodium sont résumés dans le tableau III ci-dessous.

**TABLEAU III - DIFFERENCIATTON VOIE BILIAIRE/HEPATOCYTAIRE**

| | Cellules HepaRG | Cellules HepaRG | Cellules HepaRG |
|---|---|---|---|
| | | DMSO2% | Butyrate de sodium |
| | avant traitement | Différenciation voie hépatocytaire | Différenciation voie biliaire |
| γ-Glutamyl Transférase | + | -/+ | ++ |
| Chaîne α6 | + | - | + |
| Cytokératine 7 | + | - | + |
| Cytokératine 19 | ++ | - | ++ |
| Albumine | -/+ | ++ | -/+ |
| Chaîne α1 | ++ | + | -/+ |

| | | | |
|---|---|---|---|
| -/+ : faiblement positif ; + positif ; ++ : très positif | | | |

### b/ Différenciation vers la voie pancréatique (figure 13B)

Comme l'illustre le schéma présenté en figure 1, les cellules souches "ovales" du foie sont pluripotentes. Outre qu'elles peuvent se différencier en cellules hépatiques, ce qui **est** démontré ci-dessus, elles ont des capacités de différenciation en d'autres types cellulaires dont la cellule pancréatique des acini. Nous avons recherché si ce potentiel de différenciation était une des caractéristiques de la lignée HepaRG.

Lorsque les cellules sont ensemencées et cultivées dans le milieu de culture MEM alpha, les cellules adhérent puis se rétractent pour former des structures sphéroïdes suggérant la formation de réseaux canaliculaires complexes, morphologiquement semblables aux cultures tridimensionnelles de cellules épithéliales pancréatiques (Keer-Conte et al., 1996, Ductal cyst formation in collagen-embedded adult human islet préparations : a means to the reproduction of nesidioblastosis in vitro, Diabetes, 45: 1108-1114).

### Exemple 5 : Infection de la lignée par le VHB

### 1. Matériels et méthodes

### Infection

La source infectieuse est constituée préférentiellement par le surnageant de cellules HepG2 du clone 2.2.15, concentré 100 fois. En effet, cette source infectieuse présente le double avantage d'être inépuisable et de qualité constante.

Les cellules utilisées pour l'infection sont des cellules ayant été cultivées selon les conditions définies dans l'exemple 1 (présence permanente de 5.10⁻⁵M d'hémisuccinate d'hydrocortisone) et ayant été maintenues pendant une semaine à confluence, puis pendant 2 semaines dans le même milieu additionné de 2% de DMSO.

Ces cellules sont incubées avec la source infectieuse, diluée 10 fois, dans le milieu de culture additionné de 4% de PEG 8000 (Sigma), pendant 20 heures à 37°C. La seconde partie de ce protocole d'infection par le VHB a été établie par Gripon et al, 1993, Virologgy, 192: 534-540*.*

Les cultures de contrôle ont été incubées avec 4% de PEG et 25% de SVF dilué dans une solution saline de tampon phosphate (PBS).

A la fin de l'incubation, les cellules sont lavées trois fois avec du milieu de culture et maintenues en présence de 2% de DMSO et 5.10⁻⁵M d'hémisuccinate d'hydrocortisone jusqu'à leur utilisation.

Des essais de neutralisation ont été effectués avant l'infection en incubant les virus avec des anticorps monoclonaux de surface de l'hépatite B (S39-10) pendant 1 heure à température ambiante.

### Détection de l'antigène de surface de virus de l'hépatite B (HbsAg)

L'antigène HBs a été détecté dans le milieu à l'aide du kit ELISA (Monalisa AgHBs plus®) des laboratoires Biorad. Les résultats sont exprimés en pg/ml de surnageant.

### Extraction de l'ADN viral et analyses

Des intermédiaires de réplication de l'ADN viral ont été isolés dans tous les lysats cellulaires. Les cellules sont récupérées après décollement à la trypsine, puis lysées à 37°C avec un tampon de lyse (10 mM de Tris-HCl pH 7.4, 0.5% de SDS, 10 ml d'EDTA pH 7.4, 10 ml de NaCl) additionné de protéinase K (200 µg/ml). L'ADN cellulaire est précipité en 12h, à 4°C avec du NaCl 1M. Le surnageant contenant l'ADN viral est alors extrait. Les particules virales complètes sont isolées du surnageant cellulaire par immunoprécipitation avec un anticorps polyclonal anti-HBs (Dako, France). Les acides nucléiques sont enfin extraits après 12h de lyse à 37°C, dans un tampon de lyse additionné d'ARNt (40 µg/ml) et de protéinase K (200 µg/ml).

Dans tout le protocole ci-dessus, l'ADN est extrait à l'aide du phénol-chloroforme et précipité par l'isopropanol.

Les acides nucléiques sont analysés par Southern blot sur un gel d'agarose 1.5%. Les marqueurs de poids moléculaire sont des fragments de restriction de l'ADN du virus de l'hépatite B (3182 et 1504 pb). L'hybridation est effectuée selon le protocole de Gripon et al ci-dessus.

### 2. Résultats

### Preuves de l'infection

L'ADN viral intracellulaire est analysé sur les 10 jours suivant l'infection.

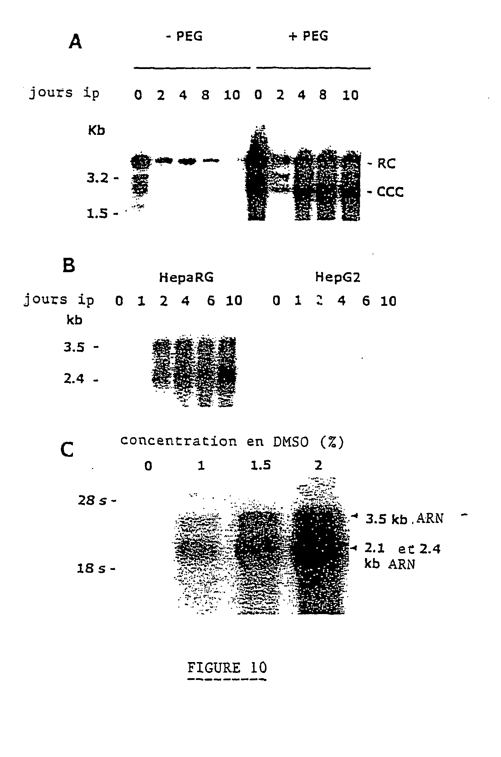

La figure 10A présente les effets du PEG sur l'infectabilité des cellules. Il s'agit d'une analyse par Southern blot de la cinétique d'apparition de l'ADN viral intracellulaire après infection des cellules HepaRG en présence de 5% de PEG (+PEG), ou en l'absence de PEG (-PEG). La position des formes d'ADN relaxé circulaire (RC) et super enroulé (CCC) sont indiquées à droite de la figure. La position de migration des marqueurs de poids moléculaire (fragments du génome du virus de l'hépatite B) est indiquée à gauche de la figure.

En l'absence de PEG, le profil d'ADN observé est identique à celui révélé pour les particules virales présentes dans l'inoculum.

Cependant, ce signal disparaît progressivement jusqu'à devenir quasi indétectable 10 jours après l'infection. De plus, aucune forme intermédiaire de réplication virale n'est détectée dans ces conditions. En revanche, celles-ci sont clairement présentes quand les cellules sont infectées en présence de PEG. Un fort signal est observé immédiatement après l'infection, avec un profil similaire à celui observé en l'absence de PEG. Ceci prouve que la pénétration des particules virales dans les cellules est effective. Au deuxième jour, le signal est fortement réduit en raison de l'élimination de nombreuses particules virales, mais on peut observer une bande fine positionnée vers 2kb. Cette bande correspond à de l'ADN ccc (ADN super enroulé clos). Les signaux détectés à des positions intermédiaires correspondent à de l'ADN viral naissant et augmentant progressivement jusqu'au 10^{ème} jour.

En parallèle, la cinétique de l'accumulation de l'ARN viral est établie. La figure 10B est une analyse par Northern blot de cette cinétique après infection des cellules HepaRG, les cellules HepG2 sont utilisées comme contrôle négatif de l'infection virale. La taille des ARNs est indiquée à gauche de la figure.

Les signaux à 2.4 et 3.5 kb, correspondant aux principales espèces d'ARN spécifiques du VHB, apparaissent après deux jours d'infection et s'accumulent seulement dans les cellules infectées.

Afin de vérifier la spécificité des cellules HepaRG à être infectées, l'infection de deux autres lignées d'hépatomes, HepG2 et BC2, a été réalisée dans des conditions identiques. Ces deux lignées ont été sélectionnées car elles sont capables de se différencier. Elles ont été cultivées dans des conditions leur permettant d'accéder à un stade de différenciation maximum, ainsi que dans les mêmes conditions que celles utilisées pour les cellules HepaRG. Après environ 12h d'incubation avec les particules virales et 5% de PEG, l'ADN viral intracellulaire et l'ARN viral produit sont analysés. Immédiatement après l'infection, une grande quantité d'ADN viral est détectée dans toutes les lignées, et surtout chez HepaRG et BC2. Dans la mesure où le traitement trypsine/EDTA élimine les particules virales adsorbées à la surface des cellules, l'ADN observé doit correspondre à des particules virales ayant pénétré à l'intérieur des cellules. Le signal décroît considérablement dans les deux jours suivant l'infection. En revanche, 18 jours après l'infection, seule la lignée HepaRG présente des formes correspondant à des intermédiaires de réplication virale ainsi, l'ADN ccc et les formes ARNs viraux. Ceci démontre que seule la lignée HepaRG est capable d'être infectée et d'initier la réplication du virus.

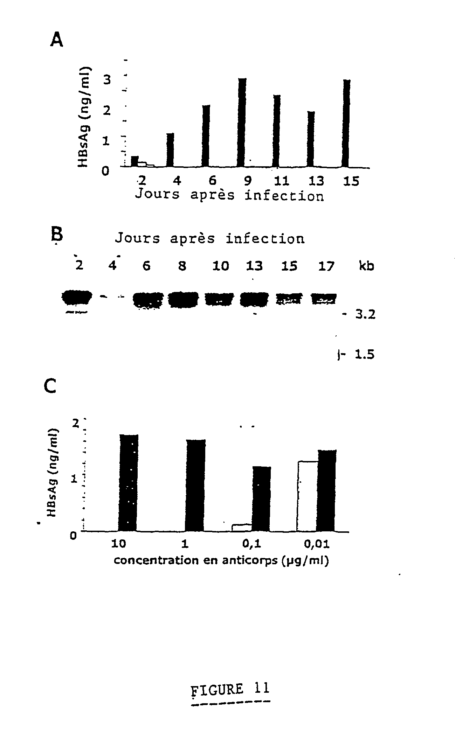

La production de particules virales a été recherchée dans le surnageant des cellules HepaRG infectées. L'antigène HBs a été dosé par radioimmunologie. La figure 11A représente une cinétique de sécrétion de l'AgHBs dans le surnageant des cellules infectées. Les surnageants ont été collectés pendant 15 jours après l'infection des cellules HepaRG (■), HBG BC2 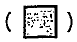 et HepG2 (□)

Une forte concentration d'antigène HBs a été détectée dans le surnageant. La concentration d'antigène a augmenté durant les 9 jours suivant l'infection, puis s'est maintenue à un niveau élevé. En revanche, dans le surnageant des cellules HepG2 et BC2, l'antigène n'a été détecté que dans les deux jours suivant l'infection, ce qui montre que les particules virales ont été adsorbées par les cellules, puis peu à peu relâchées dans le milieu.

De plus, les particules virales complètes présentes dans le milieu ont été estimées par immunoprécipitation avec un anticorps anti HBs, suivie d'une analyse par *Southern blot* de l'ADN présent dans le précipité. La figure 11B présente cette analyse par Southern blot de la cinétique d'apparition de l'ADN viral extracellulaire dans le surnageant des cellules HepaRG infectées par le VHB. Après immunoprécipitation des particules virales complètes avec un anticorps anti HBs, l'ADN viral est extrait, puis analysé. La position de migration des marqueurs de poids moléculaire (fragments du génome du virus de l'hépatite B) est indiquée à droite de la figure.

Un fort signal est observé deux jours après l'infection. Au 4^{ème} jour, il a fortement diminué mais il ré-augmente peu à peu jusqu'au 8^{ème} jour et se maintient durant toute la période de culture. Cette analyse cinétique est en adéquation avec le mode de réplication du virus : après une période de relargage des particules virales ayant pénétré la cellule (pendant 2 à 4 jours), le virus se met à répliquer activement. Le profil observé diffère légèrement de celui obtenu au 2^{ème} jour.

### Neutralisation in vitro de l'infection

Afin de déterminer si un anticorps monoclonal anti-S spécifique est capable de neutraliser l'infection des cellules, des virions sont incubés avec des concentrations variables d'anticorps, puis mis en contact avec les cellules. L'infection résiduelle est estimée en fonction de la sécrétion d'antigène HBs à long terme.

La figure 11C présente ce test de neutralisation virale *in vitro*. Les particules virales sont incubées avec des dilutions sériées d'un anticorps monoclonal dirigé contre l'antigène HBs (anticorps S 39-10) 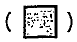 ou d'un anticorps non relevant (■) et leur infectivité évaluée sur les cellules HepaRG. Le niveau d'infection de ces cellules est estimé par mesure de la sécrétion d'antigène HBs dans le surnageant des cellules infecté, 10 jours après l'infection.

Sur cette figure, on peut voir que des concentrations de 10 et 1µg/mL bloquent l'infection virale, tandis que des concentrations inférieures ne font qu'induire une inhibition partielle. Une inhibition de 50% correspond à une concentration de 0,03 µg/mL.

### Influence du degré de différenciation sur l'infection

La capacité des cellules HepaRG à être infectées a été étudiée en fonction de leur degré de différenciation. A l'aide des méthodes décrites dans les exemples 1 et 2, le programme de différenciation des cellules a été modifié.

Une première série d'infection a été effectuée sur des cellules continuellement entretenues en présence de corticoïde seulement, puis en présence de corticoïde et de différentes concentrations de DMSO, afin d'induire différents stades de différenciation. Les analyses effectuées 2 semaines plus tard sur l'ARN viral montrent une corrélation entre l'accumulation des deux principaux transcripts viraux et le niveau de différenciation. La figure 10C présente les effets de la différenciation hépatocellulaire sur l'infection par le VHB. Il s'agit d'une analyse par Northern blot des ARNs viraux intracellulaires après infection des cellules HepaRG en présence de concentrations croissantes de DMSO.

Cette corrélation a été confirmée par une série d'expériences effectuées sur des cellules infectées à confluence (avant leur différenciation) puis placées dans des conditions optimales pour induire leur différenciation. Les résultats sont donnés dans le Tableau IV ci-dessous :

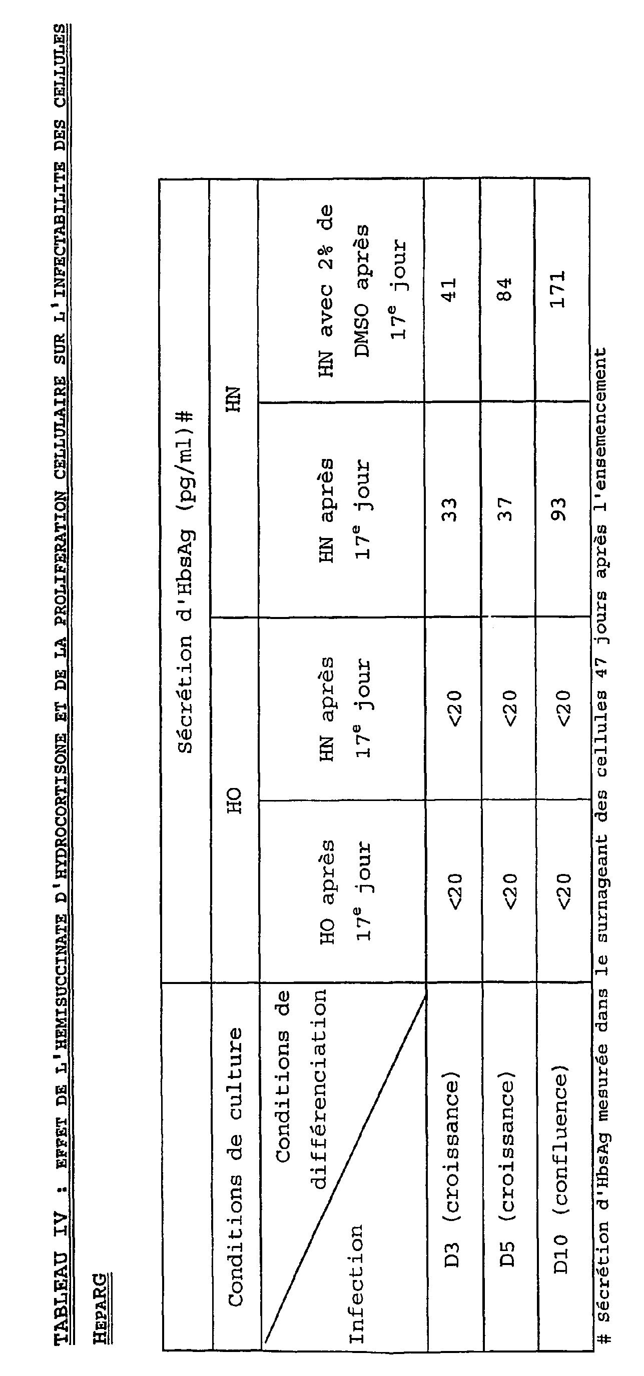

L'examen de ce tableau montre que ces quantités d'antigène HBs mesurées sont faibles lorsque les cellules sont infectées avant le traitement par le DMSO, ce qui signifie qu'une infection menée sur des cellules peu différenciées est peu efficace comparée à celle menée sur des cellules fortement différenciées. De même, une infection effectuée sur des cellules entretenues dans un milieu privé de corticoïde (pendant 10 passages), puis cultivées durant deux semaines en présence ou non de DMSO, se révèle aussi peu efficace, en particulier, pour les cellules n'ayant pas bénéficié de l'action du DMSO.

Enfin, on a comparé l'efficacité d'une infection réalisée sur des cellules indifférenciées se multipliant activement, à celle sur des cellules à croissance arrêtée et bien différenciées. Les résultats sont donnés dans le Tableau V suivant :

**TABLEAU V: EFFET DE LA DIFFERENCIATION CELLULAIRE SUR L'INFECTABILITE DES CELLULES HEPARG**

| | Sécrétion d'HbsAg (pg/ml)# | |
|---|---|---|
| Milieu de différenciation (après D17) | Infection D15 avant différenciation | Infection D28 après différenciation |
| DMSO 0% | <20 | 31 |
| DMSO 1% | 70 | 214 |
| DMSO 2% | 79 | >2000 |

| | | |
|---|---|---|
| # Sécrétion d'HbsAg mesurée dans le surnageant des cellules 47 jours après l'ensemencement | | |

Les résultats montrent qu'une infection effectuée sur des cellules en phase de prolifération est inefficace, même en présence de corticoïde et de DMSO.

### Exemple 6 : Utilisation de la lignée HepaRG pour l'évaluation de l'activité antivirale de molécules chimiques ou biologiques.

### 1. Matériels et méthodes

Les cellules sont entretenues et infectées selon les protocoles des exemples 1 et 5.

Le traitement antiviral par le 3TC est administré à partir du 8^{ème} jour et jusqu'au 15^{ème} jour suivant l'infection des cellules HepaRG, aux concentrations finales de 0.1 et 10 µM dans le milieu de culture. Un renouvellement complet du milieu est effectué tous les deux jours. Les surnageants sont stockés à -20°C et les culots cellulaires à -80°C.

L'antigène viral (HBs) a été détecté dans le surnageant de culture en utilisant le test ELISA commercial ETI-MAK-3® (SORIN) .

L'ADN viral présent dans le surnageant de culture a pu être détecté et quantifié par le test Amplicor-Monitor® (ROCHE). Les résultats sont exprimés en nombre de particules virales/ml. Pour l'analyse de l'ADN viral intracellulaire, les cellules ont été décollées à la trypsine et stockées à -80°C. L'ADN total a été isolé selon le protocole décrit précédemment (Zoulim et al, 1998, Drug therapy for chronic hepatitis B virus replication. J. Hepatol. 29:151-168).

### 2. Résultats

L'antigène HBs est détecté dans le surnageant de culture après 8 jours de traitement par le 3TC, la valeur restant constante quelle que soit la dose et égale au contrôle non traité.

Le test Amplicor-Monitor® met en évidence une diminution dose dépendante de la quantité d'ADN viral présent dans les cellules HepaRG infectées par le VHB après 8 jours de traitement par le 3TC, par rapport aux cellules de contrôles infectées par le VHB et non traitées comme le montrent les résultats du Tableau VI ci-dessous.

**TABLEAU VI - ACTIVITE ANTIVIRALE DU 3TC**

| 3TC Concentration µM | Inhibition % |
|---|---|
| 10 | 100 |
| 1 | 98 |
| 0,1 | 69 |

### Exemple 7 : Utilisation de la lignée HepaRG pour l'évaluation du pouvoir antiseptique de produits susceptibles d'inactiver le VHB, par exemple de l'eau de Javel.

### 1. Matériels et Méthodes

### 1.1. Les cellules HepaRG sont entretenues selon les protocoles de l'exemple 1.

### 1.2. L'infection est conduite en parallèle :

a) soit avec un inoculum natif obtenu comme précédemment décrit à partir des cellules HepG2 2.2.15 dont l'infectivité est documentée selon le protocole opératoire de l'exemple 5,
b) soit avec le même inoculum mis en contact avec la solution inactivante à tester, ici l'eau de Javel.

Le pouvoir antiseptique va dépendre de la concentration et du temps de contact du produit avec l'inoculum. Nous avons testé l'eau de Javel à 2 concentrations : 12° et 24° et deux durées d'exposition: 15s et 30s comme détaillé au paragraphe suivant.

Exposition du VHB de l'inoculum à l'eau de Javel.
- série 1 : javel 12° pendant 15 secondes
- " 2 : javel 12° pendant 30 secondes
- " 3 : javel 24° pendant 15 secondes
- " 4 : javel 24° pendant 30 secondes
- " 5 : H₂0 distillée utilisée pour diluer le virus : témoins virus

200 µl de stock de virus pur puis 200 µl d'eau de Javel sont versés dans des tubes de 4 ml. La réaction est arrêtée par addition de 1.6 ml de PBS (dilution au 1/10).

Elimination de l'excès de javel.

La préparation virale ci-dessus est transvasée dans des tubes spéciaux (2 tubes par série) et ultracentrifugée à 4°C de façon à ce que le VHB soit précipité dans le culot.

Le surnageant contenant l'eau de Javel est aspiré délicatement. Le petit volume résiduel des 2 tubes pour chaque dilution est mélangé et les culots de virus récupérés par homogénéisation.

La suspension virale plus ou moins inactivée de chaque série est inoculée sur les cultures de cellules HepaRG selon les conditions opératoires identiques à l'exemple 5.

### 1.3. Afin de mettre en évidence l'infection, on va mesurer à la fois les protéines et l'ADN viral.

L'Ag viral (Ag Hbs) est recherché par un test ELISA commercial (par exemple, ETI-MAK-3® de chez Sorin ou encore Monolisa Ag HBs plus® de chez Biorad) comme détaillé dans les exemples 5 et 6.

L'ADN viral est recherché en PCR dans le surnageant de culture et peut être aussi quantifié par le test Amplicor-Monitor® (ROCHE). Les résultats sont exprimés en équivalent de genome viral/ml. Pour analyser l'ADN viral intracellulaire, les cellules sont décollées à la trypsine et stockées à -80°C. L'ADN total est isolé et la recherche d'ADN spécifique du VHB est effectuée selon le protocole précédemment décrit (Zoulim, 1998).

### 2. Résultats

2.1. L'inoculum contrôle sans eau de Javel permet d'obtenir une production d'Ag Hbs et d'ADN viral dans le surnageant ainsi que les profils caractéristiques de l'ADN du VHB dans les cellules, comme détaillé dans l'exemple 5. Cette infection a été obtenue avec le virus pur et dilué au 1/10.

2.2. Avec l'inoculum exposé à différentes concentrations d'eau de Javel comme décrit ci-dessus, il apparaît que, après un contact de 15 ou 30 secondes avec de l'eau de Javel à 12°, aucune infection n'a lieu. Il en va de même avec l'eau de Javel à 24° (15, 305) et ceci pour une dilution du virus au 1/10.

### 3. Conclusions

Le test sur cellules HeparG permet donc pour la première fois d'offrir un modèle cellulaire d'infection in vitro du VHB susceptible de valider des procédures physiques (chauffage, irradiation) ou chimiques (solutions détergentes/antiseptiques ou gaz : peroxydes ozone...) capables d'inactiver ce virus contaminant majeur impliqué dans les infections nosocominales via les dispositifs et instruments médico-chirurgicaux.

Dans cet exemple, une seule dilution a été testée. Des expériences en cours préciseront la sensibilité du modèle et le nombre de logs de doses infectieuses (exprime en logarithme de base 10) qu'il est possible d'évaluer en incluant les capacités de concentrations du virus par ultracentrifugation/filtration/précipitation.

### Exemple 8 : Construction d'une "puce d'ADNc" à partir de la lignée HepaRG

Cette construction comporte les étapes suivantes :
- Préparation de deux pools d'ARN totaux, puis purification d'ARN poly A à partir d'une part, de cellules issues de la lignée différenciée HepaRG après culture dans un milieu contenant 10⁻⁵ M d'hémissuccinate d'hydrocortisone puis 2% de DMSO, et d'autre part de cellules non différenciées prélevées 5 jours après repiquage.
- Préparation des ADN complémentaires, par transcriptase inverse.
- Réalisation d'une hybridation suppressive soustractive utilisant un kit commercial.
- Clonage des ADNc présents dans la banque soustractive.
- Test de la représentativité de la banque par séquençage d'un nombre limité d'ADNc.
- Amplification par PCR des produits des ADNc d'intérêt en utilisant les amorces universelles présentes dans le vecteur.
- "Spottage" des produits de PCR selon un des procédés de type "microarray".

Ainsi l'expression de 1000 à 2000 gènes représentatifs d'états fonctionnels de l'expression de cellules hépatiques pourra être étudiée dans diverses situations d'intérêt.

### Exemple 9 : Inoculation et culture des formes hépatiques de Plasmodium falciparum dans la lignée HepaRG.

Il y a quelques années, il a été démontré que les hépatocytes humains normaux en culture primaire représentaient un système favorable à la survie du *Plasmodium falciparum* et que celui-ci pouvait supporter le cycle hépatique complet du parasite. Par contre, les modèles de réplication dans les cellules d'hépatome ont jusqu'à ce jour fait défaut.

La nouvelle lignée d'hépatome HepaRG peut représenter une avancée importante, du fait de l'observation dans un essai d'infestation de ces cellules par le parasite, d'un cycle complet avec formation de schizontes.

### Matériels et méthodes

Les sporozoïdes de *P-falciparum* sont préparés à partir de moustiques *Anophèles stephensi* infectés par ingestion de cellules sanguines, elles-mêmes infectées par les formes érythroïdes du parasite, les gamétocytes.

Deux semaines environ après le repas infectieux, les glandes salivaires infectées sont disséquées dans des conditions d'asepsie et recueillies dans du milieu de culture.

Ces suspensions, soit 10 paires de glandes salivaires par 100 ml de milieu, sont ajoutées aux cultures de cellules HepaRG.

Les cellules utilisées pour l'infestation ont été cultivées selon les conditions définies dans l'exemple 1 (présence permanente de 5.10⁻⁵ M d'hémisuccinate d'hydrocortisone) et maintenues pendant une semaine à confluence, puis pendant 2 semaines dans le même milieu additionné de 2% de DMSO.

Au moment de l'infestation, elles sont confluentes et complètement différenciées par le traitement corticoïde/DMSO décrit dans les exemples 1 à 4.

La période d'exposition ou d'inoculation est d'environ 3-4 h et effectuée dans le même milieu carencé en DMSO. Ensuite, du milieu est ajouté aux cultures et renouvelé tous les 2-3 jours.

### Détection des parasites

Les préparations sont lavées en PBS puis fixées et colorées par la coloration de May-Grunwald Giemsa. Les parasites intra-cellulaires ont une localisation cytoplasmique. La formation caractéristique de schizontes apparaît clairement. Ces schizontes présentent tout d'abord un petit nombre de noyaux, puis ils grossissent, avec un nombre de noyaux largement augmenté, attestant de la réalisation d'un processus de réplication complet.

### Optimisation du protocole d'infestation

Le *plasmodium falciparum,* parasite très fragile, est apparu très sensible au DMSO. Les résultats montrant une plus grande efficacité ont été obtenus en retirant le DMSO du milieu de culture durant la période d'inoculation.

### Exemple 10 : Infection de cellules hépaRG à une souris

Il est possible d'injecter des cellules hépaRG à un animal de laboratoire immunodéprimé tel que la souris nude afin d'obtenir un modèle d'étude *in vivo.*

Cette injection dans la souris permet aux cellules hépaRG de trouver un environnement favorable à leur prolifération et à leur différenciation. Ceci conduisant à la formation d'une masse importante de cellules différenciées. Compte-tenu du caractère pluripotent des cellules hépaRG, ces cellules subiront des différenciations différentes suivant le site d'implantation des cellules (cellules hépatiques, pancréatiques ...). Cette injection permet ainsi de reconstruire un organe de type hépatique ou pancréatique chez un animal présentant une altération dudit organe (voir article de M. Dandry et al, 2001, 33:981-988, Hepatology, Repopulation of mouce liver with human hepatocytes and in vivo infection with hepatitis B virus).

Ce modèle permet non seulement l'étude des fonctions de l'hépatocyte humain dans un organisme animal entier mais aussi d'étudier l'infection de cellules hépatiques humaines par des virus et/ou des parasites hépatotropes au sein d'un modèle animal.

### Exemple 11: Infection de la lignée par le VHC

### 1. Matériels et méthodes

### Infection

Les sources infectieuses sont issues de sérums de patients infectés par le VHC. Il s'agit à l'heure actuelle de l'unique source de virus disponible sachant qu'aucun modèle de production de virus n'a pu être développé jusqu'à ce jour. Cinq sérums ont été testés. Les virus de deux sérums (n° 2 et 4) ont un génotype 1b et celui du sérum n° 3 est un virus de génotype 3. Le génotypage n'a pas été effectué sur deux sérums utilisés, il s'agit des sérums n° 1 et 5.

Les cellules utilisées pour l'infection ont été cultivées selon les conditions définies dans l'exemple 1 (présence permanente de 5.10⁻⁵ M d'hémisuccinate d'hydrocortisone) et maintenues pendant une semaine à confluence, puis pendant 2 semaines dans le même milieu additionné de 2% de DMSO.

Ces cellules sont incubées pendant 48 heures à 37°C avec la source infectieuse, diluée 10 fois, dans le milieu de culture dépourvu de SVF et additionné de façon facultative de PEG 8000 (Sigma).

Pour contrôles, des cultures ont été incubées dans des conditions identiques mais le sérum utilisé est issu cette fois d'un patient non infecté par le VHC .

A la fin de l'incubation, les cellules sont lavées trois fois avec du milieu de culture et maintenues en présence de 2% de DMSO et 5.10⁻⁵ M d'hémisuccinate d'hydrocortisone jusqu'à leur utilisation.

### Extraction des ARN viraux intra et extracellulaires et analyses

Les formes réplicatives des ARN viraux intracellulaires ont été isolées dans tous les lysats cellulaires. Les cellules sont récupérées après décollement à la trypsine, puis l'ARN total est extrait à l'aide du kit High Pure RNA Isolation (Roche). La quantité d'ARN total extrait est estimée par densité optique afin de standardiser les différents échantillons. La qualité et l'homogénéité des ARN sont examinées en visualisant les ARN ribosomiques (ARNr : 28S et 18S) sur un gel d'agarose à 1% coloré au bromure d'éthidium. Les ARN des particules virales sont extraits du surnageant de culture à l'aide du kit High Pure viral RNA (Roche).

Par rétrotranscription (RT), l'ADN complémentaire (ADNc) des ARN viraux de polarité positive est synthétisé à partir d'une amorce spécifique hybridant en 5' de la région qui code pour la protéine de la capside virale. Cet oligonucléotide (5'-TTTGAGGTTTAGGATTYGTGCTCAT-3') dérive de celui décrit par Martell et al, 1999, Journal of Clinical Microbiology, 37 : 327-332*,* désigné C-342 . L'ADNc est ensuite amplifié par la technique de "Polymerase Chain Reaction" (PCR) en utilisant deux amorces hybridant dans la région 5' non traduite du génome viral. Les amorces utilisées sont les suivantes : l'oligonucléotide sens (5'-TGAGTGTCGTRCAGCCTCC-3'), et l'oligonucléotide antisens (5'-ACCACAAGGCCTTTCGCRACCCAC-3') qui correspond à celui conçu par Mercier et al, 1999, Journal of Virological Methods, 77 : 1-9*,* nommé NCR-3. Le produit des amplifications (amplicon de 190 pb) est visualisé sur un gel d'agarose 2% coloré au bromure d'éthidium. Un marqueur de poids moléculaire d'ADN (M) est également déposé afin de vérifier l'identité de l'amplicon par sa taille. L'efficacité des différentes étapes est toujours contrôlée en détectant l'ARN viral présent dans un standard (T+), qui correspond à un sérum dilué, issu d'un patient infecté. L'absence de toute contamination est également systématiquement vérifiée aux stades de la rétrotranscription (RT) et de l'amplification (PCR) en substituant les échantillons par de l'eau (T-).

### 2. Résultats

### Preuve de l'infection

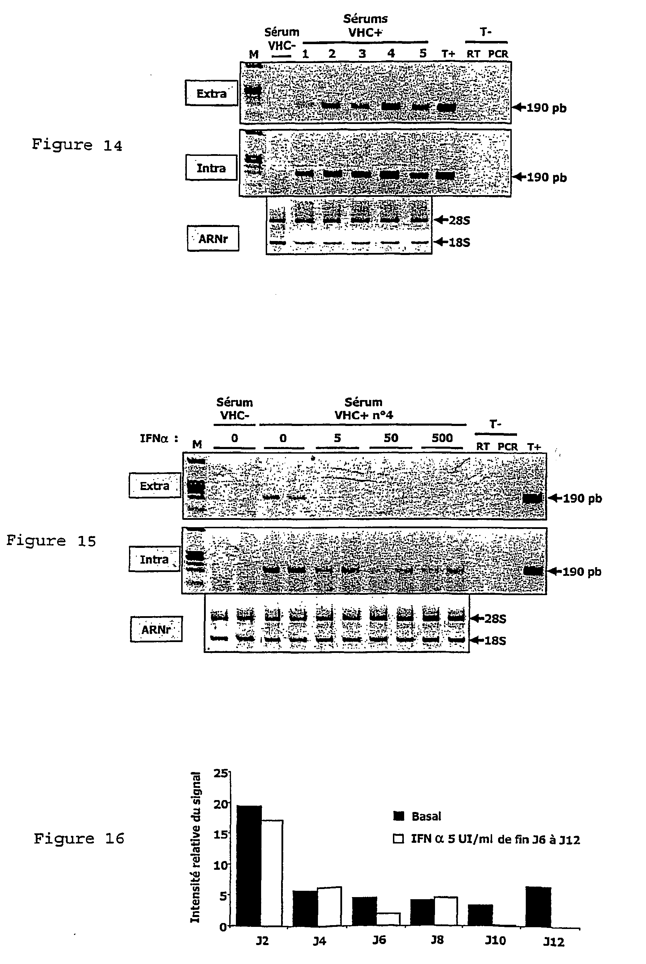

La figure 14 présente l'analyse de l'infectabilité des cellules HepaRG par des virus issus des cinq sérums testés. Il s'agit d'une recherche de la présence de l'ARN viral à la fois dans les compartiments extracellulaire et intracellulaire par RT-PCR après infection des cellules en présence de PEG 8000. L'ARN viral est recherché au 12^{ème} jour qui suit l'infection. La position de l'amplicon est indiquée à droite de la figure. Les ARNr sont montrés après homogénéisation des échantillons dans la partie inférieure de la figure où la position des ARN 28S et 18S est indiquée sur la droite.

Lors de l'utilisation d'un inoculum dépourvu de virus (sérum VHC-), aucun ARN viral n'est détecté aussi bien dans les surnageants de culture que dans les cellules. Malgré l'origine de la lignée qui a été sélectionnée à partir d'une tumeur de foie prélevée chez une patiente atteinte d'une hépatite virale C, aucune réplication virale résiduelle n'est détectable dans les HepaRG.

La technique semi-quantitative de détection par RT-PCR permet de révéler la présence de signaux non seulement dans le compartiment intracellulaire mais aussi dans les surnageants de cultures quelque soit le sérum utilisé (sérums n° 1 à 5). Ces résultats traduisent l'établissement d'une réplication du VHC dans les cellules HepaRG qui aboutit à une sécrétion efficace de virions par les cellules.

Pour confirmer qu'une réplication virale est établie dans les cellules HepaRG après leur infection, la sensibilité de la réplication du VHC à un antiviral, l'interféron α, a été étudiée. Les cellules ont été inoculées pendant 48 heures en présence de PEG 8000 par le sérum n° 4 contenant du virus. Un sérum dépourvu de VHC est utilisé comme contrôle négatif de l'infection virale. La cytokine (Introna^{®}, Schering-Plough, France) est ajoutée au milieu de culture à partir de la fin du 6^{ème} jour et ce jusqu'au 12^{ème} jour suivant l'infection des cellules, aux concentrations finales de 5, 50 ou 500 U.I. / ml. Un renouvellement complet du milieu est effectué tous les deux jours. Les surnageants sont stockés à -80°C pour l'analyse d'une cinétique.

La figure 15 montre l'incidence de la présence de l'interféron α au 12^{ème} jour qui suit l'exposition des cellules à la source infectieuse, sur la quantité d'ARN viral détectable dans les cellules et dans les surnageants. La position de l'amplicon est indiquée à droite de la figure. Les ARNr sont montrés après homogénéisation des échantillons dans la partie inférieure de la figure où la position des ARN 28S et 18S est indiquée sur la droite. Dès la plus faible dose (5 U.I. / ml), une chute de plus de 60 % du signal intracellulaire est décelée après les six jours de traitement et une disparition complète du signal est obtenue dans le milieu extracellulaire.

### Cinétique de réplication du VHC et de son inhibition par l'interféron α

Une cinétique de la réplication du VHC et de son inhibition par l'interféron α a été établie et est présentée par la figure 16. La présence de l'ARN viral dans les surnageants a été recherchée en parallèle dans une culture de référence non traitée (v) et dans une culture où la cytokine est présente dans le milieu à 5 U.I. / ml, concentration dont l'efficacité a été précédemment vérifiée (Figure 15), à partir de la fin du 6^{ème} jour qui suit l'infection et ce jusqu'au 12^{ème} jour après l'exposition à la source infectieuse (o) .

L'analyse du prélèvement effectué à la fin du 2^{ème} jour qui suit l'infection (v, J2) révèle un fort signal. Ce phénomène doit correspondre à un relargage des virus de l'inoculum qui ont été dans un premier temps adsorbés sur, ou internalisés dans, les cellules. Ensuite, le signal constant détecté dès le 4^{ème} jour post-infection (v, J4 à J12) traduit une réplication active du virus qui aboutit à une sécrétion constante de virions sur la période étudiée. La première partie de la cinétique effectuée sur les cultures incubées en présence d'interféron α est identique à celle réalisée en absence de l'antiviral (comparer ν et o de J2 à J8). Ceci permet d'affirmer qu'une réplication a bien été établie dans les cellules HepaRG avant le traitement. Par contre, après quatre jours d'incubation avec la cytokine, aucun ARN viral extracellulaire n'est détecté et ce jusqu'à la fin du traitement (o, J10 à J12). L'action inhibitrice de l'interféron α observée *in vivo* est donc bien reproduite dans les cellules HepaRG infectées par le VHC et ce après un temps de traitement minimum de 2 jours.

### Exemple 12 : Infection de la lignée par les parasites du genre Leishmania

Les leishmanioses sont des maladies secondaires à l'infection par un parasite du genre *leishmania.* La présentation clinique de ces infections va de l'infection cutanée localisée, à une infection disséminée au cours de laquelle les principaux organes infectés sont les ganglions, la moelle osseuse, la rate et le foie. Les leishmanies sont transmises sous forme promastigote flagellé par un diptère hématophage, le phlébotome, et deviennent rapidement intracellulaires chez leur hôte. Les cellules actuellement décrites comme permissives aux leishmanies sont les cellules du système des phagocytes mononucléés. Toutefois, un essai d'infection d'hépatocytes a récemment mis en évidence la permissivité de ce type cellulaire, ouvrant des perspectives de recherches afin de mieux comprendre la physiopathologie de l'infection et d'évaluer de nouvelles cibles thérapeutiques.

### 1. Matériels et méthodes

### Infection

Les promastigotes de *Leishmania major, L. donovani, L. infantum, L. tropica*, *L. braziliensis* et *L. guyanensis* utilisées sont des souches isolées de patients, identifiées par la méthode isoenzymatique de référence et cryopréservées dans l'azote liquide. L'obtention d'un inoculum nécessite au moins 2 phases d'amplification successives en milieu gélosé Novy-McNeal-Nicolle additionné de sang de lapin puis en milieu de Schneider additionné de 10% de SVF.

Les cellules utilisées pour l'infection ont été cultivées selon les conditions définies dans l'exemple 1 (présence permanente de 5.10⁻⁵ M d'hémisuccinate d'hydrocortisone) et maintenues pendant une semaine à confluence, puis pendant 2 semaines dans le même milieu additionné de 2% de DMSO.

Ces cellules sont incubées pendant 18 heures à 37°C avec les promastigotes de leishmanies, puis lavées 3 fois avec du milieu de culture et maintenues en présence de 2% de DMSO et 5 M d'hémisuccinate d'hydrocortisone.

### Détection des parasites intracellulaires

Le contrôle et la quantification de l'infection se font par microscopie optique. Les cellules sont récupérées après trypsinisation des puits et 2 lavages en PBS, cytocentrifugés 10 minutes à 9000 tours/minute, puis fixées et colorées par la coloration de May-Grunwald Giemsa. Les parasites intracellulaires sous forme amastigote ont une localisation cytoplasmique, mesurent 3x6 micromètres et sont facilement repérables grâce à leur structure comprenant un cytoplasme bleuté, et un noyau et un kinétoplaste violets. Le nombre de cellules infectées et le nombre total de parasites retrouvés sont calculés pour un nombre moyen de 4000 hépatocytes lus.

### 2.Résultats

### Preuve de l'infection

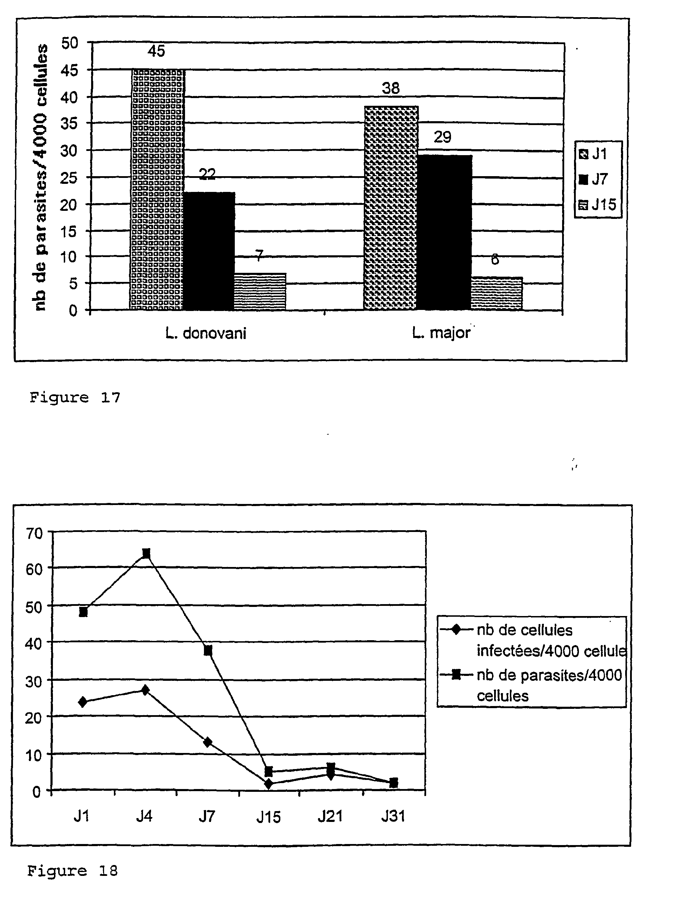

La figure 17 présente les cinétiques d'infection observées avec une souche de *L. donovani* et une souche de *L. major* pour des ratios d'infection de 25 parasites pour une cellule. Les cellules sont donc infectables avec une souche hépatotrope (*L. donovani)* mais aussi une souche dermatrope (*L*. *major) .* Le nombre d'amastigotes de *L. donovani* pour 100 cellules est compris entre 7 et 45 pour 4000 cellules lues (0,175% à 1,125%), correspondant à un pourcentage de cellules infectées compris entre 0,1 à 0,55 %. Le nombre d'amastigotes de *L. major* est compris entre et 6 et 38 pour 4000 cellules lus (0,15 et 0,725 %), correspondant à un pourcentage de cellules infectées compris entre 0,1 à 0,375 %.

### Optimisation du protocole de l'infection

Une première expérimentation a étudié l'effet "durée de culture" sur le nombre total de parasites et le nombre de cellules infectées. Les résultats de ce travail effectué avec des cellules infectées par *L*. *major* (ratio d'infection = 25 parasites pour 1 cellule) sont présentés dans la figure 18.

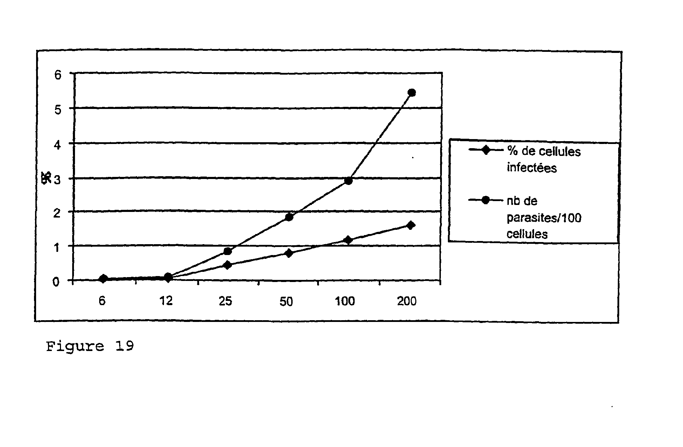

Une seconde expérimentation visait à étudier l'effet "inoculum" sur le niveau d'infection des cellules. *L.* major a successivement été inoculé aux cellules avec des ratios de 6, 12, 25, 50, 100 et 200 parasites pour 1 cellule. Les résultats des cultures à J7 sont présentés dans la figure 19 et montrent que le nombre de parasites pour 100 cellules variait de 2 à 218 pour 4000 cellules lues (0,05% à 5,45%), correspondant à un pourcentage de cellules infectées allant de 0,05% à 1,6%.

Au total, il apparaît que le ratio d'infection idéal par *L*. *major* est de 100 parasites pour 1 cellule, et que la quantification de l'infection parasitaire est optimale entre J4 et J7.

## Revendications

1. Lignée cellulaire d'hépatomes telle que déposée le 5 avril 2001 à CNCM, sous le n°I-2652.

2. Procédé d'infection de cellules hépatiques par un parasite hépatotrope et/ou un virus, avec une phase de sélection comportant une phase de prolifération cellulaire dans un milieu de culture comportant en continu au moins un cortico-stéroïde à une concentration non toxique qui favorise la différenciation des hépatocytes humains normaux, puis, la confluence atteinte, une phase de différenciation cellulaire dans ce même milieu, par addition de DMSO, en quantité suffisante pour induire la différenciation,
ce procédé étant réitéré plusieurs fois, préférentiellement 3 fois, si souhaité,
ledit procédé étant **caractérisé en ce qu'**il comporte
- une phase de différenciation permettant aux cellules d'atteindre une morphologie proche de l'hépatocyte à l'aide du milieu de culture comportant au moins un cortico-stéroïde à une concentration non toxique qui favorise la différenciation des hépatocytes humains normaux, additionné de DMSO, en quantité suffisante pour induire une différenciation,
- une phase d'infection avec incubation des cellules hépatiques avec le parasite hépatotrope et/ou un virus, dans un milieu de culture comportant au moins un cortico-stéroïde à une concentration non toxique qui favorise la différenciation des hépatocytes humains normaux, additionné de la source infectieuse.

3. Procédé selon la revendication 2, **caractérisé en ce que** les milieux utilisés comportent de l'insuline, à raison de 2,5 µg/ml à 10 µg/ml pour favoriser la survie des hépatocytes humains normaux.

4. Procédé de transfection de la lignée selon la revendication 1 à l'aide d'un vecteur comportant la séquence complète et/ou une partie du matériel génétique des virus VHB et/ou VHC.

5. Procédé de criblage à haut débit de gènes différentiellement exprimés à l'aide d'un outil de type puce réalisée à partir de la lignée selon la revendication 1, ces gènes étant différentiellement exprimés sous l'effet de conditions de culture, de l'exposition à une molécule et/ou un virus et/ou un parasite.

6. Utilisation de la lignée cellulaire selon la revendication 1, pour des tests métaboliques et/ou de toxicité destinés à l'évaluation de nouveaux médicaments et/ou de constituants nutritionnels et/ou de polluants de l'environnement.

7. Utilisation de la lignée cellulaire selon la revendication 1, pour la fabrication d'un bioréacteur extracorporel pour traiter transitoirement les insuffisances hépatocellulaires aigües.

8. Utilisation de la lignée cellulaire selon la revendication 1, pour le criblage et/ou la fabrication de nouveaux vaccins et/ou de molécules antivirales, en particulier pour le criblage de molécules actives vis-à-vis de l'une des étapes du cycle viral.

## Claims

1. A human hepatoma cell line as deposited on 5th April 2001 at the CNCM under No. 1-2652.

2. A method for infecting hepatic cells with a hepatotropic parasite and/or a virus, with a selection phase comprising a phase of cell proliferation in a culture medium comprising continuously at least one cortico-steroid at a non-toxic concentration which promotes the differentiation of normal human hepatocytes, then, upon reaching confluence, a phase of cell differentiation in this same medium, by adding DMSO, in a quantity sufficient to induce differentiation, said process being repeated several times, preferably 3 times, if desired, said method being **characterized in that** it comprises
- a differentiation phase allowing the cells to reach a morphology close to the hepatocyte using culture medium comprising at least one cortico-steroid at a non-toxic concentration which promotes differentiation of normal human hepatocytes, with DMSO added to it in a quantity sufficient to induce differentiation,
- an infection phase with incubation of the hepatic cells with a hepatotropic parasite and/or a virus in a culture medium comprising at least one cortico-steroid at a non-toxic concentration which promotes differentiation of normal human hepatocytes, to which the infections source is added.

3. The process according to claim 2, wherein the used medina contain insulin, at the rate from 2.5 µg/ml to 10 µg/ml of insulin to promote the survival of normal human hepatocytes.

4. A method for transfection of the cell line according to claim 1 with a vector comprising the complete and/or partial sequence of the genetic material of HBV and/or HCV virus.

5. A method for high flow rate screening of differentially expressed genes using a chip-type tool produced from the cell line according to claim 1, said genes being differentially expressed under the effect of culture conditions, exposition to a molecule and/or a virus and/or a parasite.

6. The use of the cell line according to claim 1, for metabolic and/or toxicity tests intended for the evaluation of new medicaments and/or nutritional constituents and/or environmental pollutants.

7. The use of the cell line according to claim 1, for making an extracorporeal bioreactor for the transient treatment of acute hepatocellular insufficiencies.

8. The use of the cell line according to claim 1, for screening and/or manufacturing new vaccines and/or antiviral molecules particularly, for the screening of active molecules with respect to one the steps of the viral cycle.

## Patentansprüche

1. Hepatomzelllinie, wie sle am 5. April 2001 bei der CNCM unter der Nr. 1-2652 hinterlegt wurde.

2. Verfahren zur Infektion von Leberzellen mit einem hepatotropen Parasiten und/oder einem Virus, mit einer Selektionsphase, die eine Phase der Zellvermehrung in einem Kulturmedium, das kontinuierlich wenigstens ein Corticosteroid in einer nichttoxischen Konzentration, das die Differenzierung von normalen humanen Hepatocyten begünstigt, umfasst, und dann, nachdem die Konfluenz erreicht ist, eine Phase der Zelldifferenzierung in demselben Medium durch Zugabe von PMSO in ausreichender Menge, um die Differenzierung zu induzieren, umfasst;
wobei dieses Verfahren gegebenenfalls mehrmals, vorzugsweise dreimal, wiederholt wird;
wobei dieses Verfahren **dadurch gekennzeichnet ist, dass** es Folgendes umfasst:
- eine Differenzierungsphase, die es den Zellen erlaubt, eine Morphologie zu erreichen, die derjenigen von Hepatocyten nahekommt, und zwar mit Hilfe des Kulturmediums, das wenigstens ein Corticosteroid in einer nichttoxischen Konzentration, das die Differenzierung von normalen humanen Hepatocyten begünstigt und das mit DMSO in ausreichender Menge, um die Differenzierung zu induzieren, versetzt ist, umfasst;
- eine Infektionsphase mit Inkubation der Leberzellen mit dem hepatotropen Parasiten und/oder dem Virus in einem Kulturmedium, das wenigstens ein Corticosteroid in einer nichttoxischen Konzentration, das die Differenzierung von normalen humanen Hepatocyten begünstigt und mit der Infektionsquelle versetzt ist, umfasst.

3. Verfahren gemäß Anspruch 2, **dadurch gekennzeichnet, dass** die verwendeten Medien Insulin in einer Menge von 2,5 µg/ml bis 10 µg/ml umfassen, um das Überleben der normalen humanen Hepatocyten zu begünstigen.

4. Verfahren zur Transfektion der Linie gemäß Anspruch 1 mit Hilfe eines Vektors, der die vollständige Sequenz und/oder einen Teil des genetischen Materials der Viren HBV und/oder HCV umfasst.

5. Hochdurchsatz-Screeningverfahren für differentiell exprimierte Gene mit Hilfe eines Hilfsmittels des Chiptyps, das ausgehend von der Linie gemäß Anspruch 1 hergestellt wird, wobei diese Gene unter der Wirkung von Kulturbedingungen, der Einwirkung eines Moleküls und/oder eines Virus und/oder Parasiten differentiell exprimiert werden.

6. Verwendung der Zelllinie gemäß Anspruch 1 für Stoffwechsel- und/oder Toxizitätstests zur Bewertung von neuen Medikamenten und/oder Nahrungsbestandteilen und/oder Umweltschadstoffen.

7. Verwendung der Zelllinie gemäß Anspruch 1 zur Herstellung eines extrakorporalen Bioreaktors zur vorübergehenden Behandlung von akutem Leberversagen.

8. Verwendung der Zelllinie gemäß Anspruch 1 zum Screening und/oder zur Herstellung neuer Impfstoffe und/oder antiviraler Moleküle, insbesondere zum Screening von Molekülen, die gegenüber einem der Stadien des viralen Zyklus aktiv sind.
